# NEUE EUROPÄISCHE PATENTSCHRIFT

(11) **EP 2 571 632 B3**
(45) Veröffentlichungstag dieser Patentschrift: **03.05.2023**
(45) Hinweis auf die Patenterteilung: 05.11.2014
(21) Anmeldenummer: 11719553.7
(22) Anmeldetag: 12.05.2011
(51) Int. Cl.: B08B 13/00, B08B 3/10, A62B 25/00, A61L 2/16, A47L 15/00, A61B 19/00, B08B 3/02, B08B 3/04, B08B 5/00, B08B 3/08

(54) **VORRICHTUNG ZUR REINIGUNG VON ATEMGERÄTEN**
DEVICE FOR CLEANING RESPIRATORS
DISPOSITIF POUR NETTOYER DES APPAREILS DE RESPIRATION

(30) Priorität: 21.05.2010 DE 102010029221
(43) Veröffentlichungstag der Anmeldung: 27.03.2013
(73) Patentinhaber: MEIKO Maschinenbau GmbH & Co. KG, 77652 Offenburg (DE)
(72) Erfinder: ACKERMANN, Frank, 77948 Friesenheim (DE); BRAUN, Markus, 77656 Offenburg (DE); PEUKERT, Thomas, 77815 Bühl (DE); RAUBER, Hans-Josef, 77784 Oberhamersbach (DE); SIMUNDIC, Marijan, 77797 Ohlsbach (DE)
(74) Vertreter: Altmann Stößel Dick Patentanwälte PartG mbB
(86) Internationale Anmeldenummer: PCT/EP2011/057706
(87) Internationale Veröffentlichungsnummer: WO 2011/144518

(56) Entgegenhaltungen:
- DE-A1- 10 020 835
- DE-B- 1 174 169
- DE-B4-102007 012 768
- DE-U1- 29 822 172
- US-A- 3 881 503
- US-A1- 2009 183 753

## Beschreibung

### Gebiet der Erfindung

Die Erfindung betrifft eine Reinigungsvorrichtung zur Reinigung und optional zusätzlich auch zur Desinfektion von Atemgeräten oder auch Bestandteilen von Atemgeräten. Weiterhin betrifft die Erfindung eine Halterung zum Einsatz in einer Reinigungsvorrichtung sowie eine Verwendung einer Reinigungsvorrichtung und ein Verfahren zur Reinigung von Atemgeräten. Derartige Vorrichtungen und Verfahren werden allgemein eingesetzt, um Atemgeräte oder Bestandteile derselben zu reinigen, beispielsweise Atemgeräte für Rettungskräfte wie Feuerwehr, technische Hilfsorganisationen oder Rettungssanitäter, Atemgeräte für Taucher oder allgemein Personen in lebensfeindlichen oder kritischen Arbeitsumgebungen sowie für Streitkräfte und Sicherheitskräfte wie beispielsweise Polizisten. Auch für Atemgeräte im medizinischen Bereich, beispielsweise Atemmasken für Sauerstoffversorgung und Operationen, lassen sich die vorgeschlagenen Vorrichtungen und Verfahren einsetzen. Insbesondere kommt allgemein die Reinigung von Atemmasken oder Lungenautomaten als Einsatzgebiet in Betracht.

### Stand der Technik

Atemgeräte wie beispielsweise Atemschutzmasken oder Lungenautomaten, sind in der Regel Bestandteil der persönlichen Schutzausrüstung beispielsweise von Rettungskräften, Streitkräften oder Sicherheitskräften. So sind aus dem Stand der Technik eine Vielzahl von Atemgeräten für unterschiedliche Einsatzzwecke bekannt. Beispielsweise verwenden Rettungskräfte wie Feuerwehren Atemschutzmasken mit Filtern zur Entfernung schädlicher Bestandteile aus der angesaugten Atemluft. In vielen Fällen wird jedoch, alternativ oder zusätzlich zu einem Filter, ein so genannter Lungenautomat verwendet, über welchen der Benutzer mit einem Atemgas, beispielsweise Pressluft, beatmet werden kann. Lungenautomaten, welche häufig auch als Atemregler bezeichnet werden, ermöglichen es allgemein einem Benutzer, aus einer Druckgasflasche oder einem anderen Druckgasanschluss zu atmen und sich auf diese Weise beispielsweise unter Wasser oder in einer anderen, nicht atembaren oder giftigen Atmosphäre aufzuhalten. Dazu wird das Druckgas, beispielsweise komprimierte Luft, aus dem Druckgasanschluss durch den Lungenautomaten auf einen in einer Arbeitsumgebung des Benutzers herrschenden Druck angepasst.

Die Atemgeräte oder Bestandteile derselben müssen in der Regel nach jedem Einsatz gereinigt, hygienisiert, getrocknet, geprüft und gegebenenfalls instandgesetzt und verpackt werden. Mit der Reinigung sollen alle Verschmutzungen infolge eines Gebrauchs oder einer Lagerung entfernt werden, so dass die Atemgeräte makroskopisch sauber und hygienisch einwandfrei bereitgestellt werden können, beispielsweise für die nächsten Schritte einer Aufbereitung. Dieselben Anforderungen gelten in der Regel auch für andere Bestandteile von Atemgeräten, wie beispielsweise Anbau- und Zubehörteile von Atemmasken, wie beispielsweise Filter oder Lungenautomaten. Da Atemgeräte bzw. deren Bestandteile in der Regel sicherheitsrelevante Vorrichtungen sind, sind bei der Reinigung dieser Vorrichtungen mehrere Anforderungen zu beachten. Neben einer ausreichenden Reinigung und Hygienisierung ist beispielsweise in vielen Fällen zu beachten, dass Zubehörteile aus technischen Gründen den jeweiligen Atemmasken zugeordnet bleiben müssen. Weiterhin besteht in der Regel die Anforderung, dass gasführende Bereiche bestimmter Elemente von Atemgeräten, beispielsweise gasführende Bereiche von Lungenautomaten, nicht mit Reinigungsfluid, beispielsweise nicht mit Wasser und/oder Reinigungslösung, in Berührung kommen dürfen.

In vielen Fällen werden Atemgeräte, wie beispielsweise Atemmasken und deren Zubehör, entweder von Hand gereinigt oder in modifizierten Wäschewaschmaschinen mit Hilfe von Schutzbeuteln und/oder mit Hilfe von Adaptern gewaschen. Beispielsweise ist aus EP 0 935 687 B1 allgemein eine Waschmaschine bekannt, welche einen Laugenbehälter mit einer Trommel aufweist. Ein Mantel der Trommel weist eine zum Trommelinneren gerichtete Wölbestruktur auf, wobei auf zum Trommeläußeren gerichteten Randkonturen der Wölbung in ihren Eckpunkten Löcher angeordnet sind. Mit derartigen Waschmaschinen sind grundsätzlich besonders schonende Reinigungen von Ausrüstungsgegenständen für Rettungskräfte möglich.

Aus EP 1 088 928 A1 ist ein Haltesystem für Atemschutzmasken in einer Wäschebehandlungsmaschine bekannt. Das Haltesystem weist einen Tragbügel auf, welcher in einer Trommel der Wäschebehandlungsmaschine mitdrehend angeordnet ist und mit welchem die Atemschutzmasken verbunden werden können.

Aus DE 200 03 743 U1 und aus DE 298 22 172 U1 sind jeweils Vorrichtungen zum Behandeln von Schutzanzügen bekannt. Dabei werden Kleiderbügel verwendet, welche flexible Luftaustrittsdüsen umfassen. Die Kleiderbügel sind jeweils an einer Schwenkvorrichtung befestigt. Eine Reinigung von Atemgeräten ist allgemein mittels der gezeigten Vorrichtungen nicht oder nur schwer möglich.

Aus DE 10 2005 033 618 B3 ist eine Vorrichtung zur Reinigung von Atemschutzmasken bekannt. Die Vorrichtung umfasst ein verschließbares Gehäuse sowie mindestens eine in einem Träger angeordnete Aufnahme für mindestens eine Atemschutzmaske. Weiterhin ist eine Düsenanordnung und eine Bürstenanordnung vorgesehen, wobei durch eine Bewegung der Atemschutzmasken eine Bebürstung der Atemschutzmasken erfolgt. Mittels der offenbarten Vorrichtung ist allerdings eine individuelle Zuordnung und Reinigung von Zubehörteilen von Masken nicht möglich. Weiterhin ist die Reinigung von gasführenden Elementen, wie beispielsweise Lungenautomaten, mit der offenbarten Vorrichtung nicht möglich. Auch aus DE 200 03 744 U1 ist eine Vorrichtung zum Reinigen, Desinfizieren und Trocknen von Atemschutzmasken bekannt, welche ein Tragegestell mit einem zugeordneten Düsensystem und einzelnen Behandlungsplätzen aufweist. Auch diese Vorrichtung ist grundsätzlich nicht zur Reinigung von gasführenden Elementen und Zubehörteilen geeignet.

Aus DE 10 2007 009 936 A1 ist eine Reinigungsvorrichtung für Pressluftatmer bekannt. Diese weist einen durch ein Schutzgitter begrenzten Aufnahmeraum sowie rotierende Düsenträger auf. Die Düsenträger befinden sich dabei außerhalb des Schutzgitters. Nachteilig an der dargestellten Vorrichtung ist jedoch, dass Reinigungsflüssigkeit in gasführende Bereiche eindringen kann.

Die Reinigung von empfindlichen Bauelementen von Atemgeräten, wie beispielsweise Lungenautomaten, erfolgt hingegen in der Regel manuell. Gegebenenfalls kann eine manuelle Reinigung durch ein Einlegen in Ultraschall-Reinigungsgeräte unterstützt werden. Aus DE 10 2007 012 768 B4 sind jedoch ein Verfahren und eine Vorrichtung zur Reinigung von Lungenautomaten bekannt. Dabei werden die zu reinigenden Gegenstände auf Halterungen eines rotierenden Elements aufgesteckt und mehrmals in ein Flüssigkeitsbart mit Reinigungsflüssigkeit, Desinfektionsflüssigkeit und Spülflüssigkeit eingetaucht. Lungenautomaten werden dabei zuerst zur Dichtsetzung zwischen Ventil und Schlauchanschluss mit Druckluft beaufschlagt und danach in das Flüssigkeitsbad eingetaucht. Nachteilig an derartigen Tauchverfahren ist jedoch, dass aufwändige Halterungen mit entsprechenden Aktoren erforderlich sind, um durch entsprechende Bewegungen eine Entfernung von Reinigungsfluid aus den verschiedenen Hohlräumen nach der Reinigung zu gewährleisten.

DE 100 20 835 A1 beschreibt eine Vorrichtung zur Behandlung von Atemschutzmasken gemäß dem Oberbegriff des Anspruchs 1. Es ist eine Aufnahme zur Bestückung mit Atemschutzmasken vorgesehen, bei welcher eine Kopplung der Atemschutzmasken mittels des Atemgeräteanschlusses an die Aufnahme erfolgt. Weiterhin wird beschrieben, dass in einer Kabine der Vorrichtung eine Sammeleinrichtung zur Sammlung von Behandlungsmitteln vorgesehen ist, sowie eine Pumpe für die Zufuhr der Behandlungsmittel.

US 3,881,503 A offenbart eine Vorrichtung zum Waschen und Dekontaminieren von Anästhesie-Ausrüstungsgegenständen. Es ist ein Düsensystem vorgesehen, durch welches unter hohem Druck Wasserstrahlen auf das Reinigungsgut aufgesprüht werden können.

In DE 11 74 169 B wird eine Vorrichtung zur Reinigung von Atemschutzmasken beschrieben. Dabei ist in einem Gehäuse ein drehbargelagertes Rohrgestell vorgesehen, bei welchem die zu reinigenden Masken auf Halterungen aufgespannt werden. Mittels des Drehgestells werden die Atemschutzmasken mit einer Drehbewegung durch ein Bad einer Reinigungsflüssigkeit in einer Wanne hindurch bewegt.

Aus dem medizinischen Umfeld sind weiterhin Spülmaschinen bekannt, in welchen Beatmungsschläuche gespült werden können. Derartige Spülmaschinen sind in der Regel als Einkreis-Maschinen, auch als Wasserwechsel-Maschinen bezeichnet, ausgestaltet. Dies bedeutet, dass die Spülmaschinen einen Spülbehälter aufweisen, in welchem die Reinigung und die Konditionierung der Reinigungsflüssigkeit erfolgen. Für den Wechsel einer Reinigungsflüssigkeit, beispielsweise für einen Wechsel von einer Waschlauge hin zu einer Klarspülflüssigkeit, ist ein vollständiger Wasserwechsel innerhalb des Behälters erforderlich.

Die bekannten Verfahren und Vorrichtungen zur Reinigung von Atemgeräten wie beispielsweise Atemschutzmasken und deren Zubehörteilen weisen eine Vielzahl von Nachteilen auf. So ist eine manuelle Reinigung der Atemgeräte sehr arbeitsintensiv. Zudem ist der Reinigungsprozess in diesem Fall stark von der einzelnen Reinigungskraft beeinflusst und lässt sich hierdurch kaum standardisieren.

Die Reinigung in modifizierten Wäschewaschmaschinen ist hingegen vergleichsweise zeitintensiv. Zudem sind die Atemgeräte oder deren Bestandteile in vielen Fällen nach der Reinigung im Inneren mit Reinigungsfluid befüllt, da diese entweder in der Trommel der Waschmaschine wahllos angeordnet sind oder da deren Stellung innerhalb der Trommel fixiert ist und nicht gezielt beeinflusst werden kann, so dass die Atemgeräte mit Beendigung der Reinigungsprozedur in der Regel nicht leerlaufen können. Zubehörteile können meist nicht in der Waschmaschine mitgereinigt werden, da während des Prozesses häufig eine individuelle Zuordnung verloren geht.

Besonders kritisch ist mit den bekannten Verfahren und Vorrichtungen die Reinigung von gasführenden Elementen der Atemgeräte wie beispielsweise einem oder mehreren Rohrleitungen, Schläuchen oder Ventilen und/oder Lungenautomaten. Für derartige Lungenautomaten stehen bislang nur wenige automatisierte Reinigungsverfahren zur Verfügung, welche den oben genannten Sicherheitsanforderungen genügen würden. Das aus DE 10 2007 012 768 B4 bekannte Reinigungsverfahren weist hingegen die oben beschriebenen Nachteile auf und ist vergleichsweise aufwändig. Weiterhin ermöglicht die dargestellte Vorrichtung nicht gleichzeitig die Reinigung und ggf. Desinfektion von Lungenautomaten und Atemschutzmasken sowie Zubehörteilen. Auch Spülmaschinen aus dem medizinischen Umfeld, welche für eine Maskenreinigung adaptiert wurden, sind nicht dafür geeignet, Lungenautomaten zu reinigen und ausreichend zu hygienisieren.

### Aufgabe der Erfindung

Es ist daher eine Aufgabe der vorliegenden Erfindung, Vorrichtungen und Verfahren zur Reinigung und optional zusätzlich zur Desinfektion von Atemgeräten bereitzustellen, welche die oben beschriebenen Nachteile vermeiden. Insbesondere soll eine zuverlässige, schnelle und dennoch sämtlichen Sicherheitsanforderungen genügende Reinigung auch von gasführenden Elementen von Atemgeräten ermöglicht werden, wie beispielsweise gasführenden Elementen mit mindestens einem internen Ventil, wie beispielsweise Lungenautomaten.

### Offenbarung der Erfindung

Diese Aufgabe wird gelöst durch Vorrichtungen und Verfahren mit den Merkmalen der unabhängigen Ansprüche. Vorteilhafte Weiterbildungen der Erfindung, welche einzeln oder in beliebiger Kombination realisierbar sind, sind in den abhängigen Ansprüchen dargestellt.

In einem ersten Aspekt der Erfindung wird eine Reinigungsvorrichtung zur Reinigung und optional zusätzlich auch zur Desinfektion von Atemgeräten vorgeschlagen. Unter Atemgeräten werden dabei allgemein Vorrichtungen verstanden, welche in irgendeiner Weise zur Bereitstellung von Atemgas an mindestens einen menschlichen und/oder tierischen Benutzer eingerichtet sind. Bei diesen Atemgeräten kann es sich dabei um vollständige, betriebsfertige Atemgeräte handeln, oder auch um Bestandteile derselben, so dass im Folgenden zwischen Atemgeräten und deren Bestandteilen begrifflich nicht unterschieden wird. Insbesondere können die Atemgeräte Atemmasken, Atemschutzmasken, Schläuche, Ventile, Filter, Druckgasbehälter, Lungenautomaten oder Kombinationen der genannten und/oder anderer Elemente umfassen.

Die Reinigungsvorrichtung umfasst mindestens eine Reinigungskammer zur Aufnahme mindestens eines Atemgeräts. Diese Reinigungskammer kann grundsätzlich als geschlossene, geöffnete oder zu öffnende Reinigungskammer ausgestaltet sein. Besonders bevorzugt ist es, wenn die Reinigungskammer allseitig oder zumindest in zwei Dimensionen von einem Gehäuse umschlossen ist, welches vollständig geschlossen ausgestaltet sein kann, welches jedoch grundsätzlich auch eine oder mehrere Durchführungen aufweisen kann. Die Reinigungskammer kann insbesondere als starre Reinigungskammer ausgestaltet sein, also als Reinigungskammer, welche während eines Reinigungsvorgangs ihre Position und/oder Ausrichtung nicht verändert, kann jedoch grundsätzlich auch als bewegliche Reinigungskammer ausgestaltet sein, beispielsweise als schwenkbare und/oder drehbare Reinigungskammer, welche während eines Reinigungsvorgangs in der Reinigungsvorrichtung ihre Position und/oder Orientierung verändert, beispielsweise durch eine Drehung, eine Rotation, einen Schleudervorgang, einen Rüttelvorgang oder ähnliche Bewegungen. Insofern kann die Reinigungskammer beispielsweise als Spülkammer einer Spülmaschine ausgestaltet sein und die Reinigungsvorrichtung als Spülmaschine, und/oder die Reinigungskammer kann als Trommel einer Waschmaschine ausgestaltet sein, und die Reinigungsvorrichtung in Form einer Waschmaschine. Beispielsweise können Geschirrspülmaschinen und/oder Wäschewaschmaschinen kommerzieller Art oder für den Haushaltsbereich erfindungsgemäß modifiziert werden.

Die Reinigungsvorrichtung weist weiterhin mindestens eine Fluideinrichtung zur Beaufschlagung des Atemgeräts mit mindestens einem Reinigungsfluid auf. Unter einem Reinigungsfluid ist dabei eine grundsätzlich beliebige Flüssigkeit zu verstehen. Unter einer Fluideinrichtung ist allgemein eine Vorrichtung zu verstehen, mittels derer das innerhalb der Reinigungskammer aufgenommene Atemgerät in beliebiger Weise direkt oder indirekt mit dem Reinigungsfluid beaufschlagt werden kann. Dies erfolgt in Form eines direkten Beaufschlagens, durch ein Besprühen, Betropfen, Bestrahlen oder eine Kombination der genannten und/oder anderer direkter Beaufschlagungsarten, bei welchen aus der Fluideinrichtung austretendes Reinigungsfluid unmittelbar auf das Atemgerät auftrifft. Diese Ausgestaltung der Beaufschlagung kann insbesondere bei fester Reinigungskammer erfolgen, beispielsweise bei einer Spülmaschine. Alternativ oder zusätzlich kann die Beaufschlagung mittels der Fluideinrichtung auch derart erfolgen, dass die Fluideinrichtung die Reinigungskammer vollständig oder teilweise mit Reinigungsfluid füllt, so dass das in der Reinigungskammer aufgenommene Reinigungsfluid zumindest in einer Stellung der Reinigungskammer mit dem Reinigungsfluid in Kontakt kommt. Diese Ausgestaltung der Beaufschlagung kann insbesondere bei einer Ausgestaltung der Reinigungskammer als bewegliche Reinigungskammer genutzt werden, beispielsweise in Form einer Trommel. Auch Kombinationen der genannten Beaufschlagungsarten und/oder anderer Beaufschlagungsarten sind möglich. Unabhängig davon, ob die Reinigungskammer starr oder beweglich ausgestaltet ist, kann die Beaufschlagung mit dem Reinigungsfluid im einfachen Betrieb erfolgen, indem das Reinigungsfluid lediglich einmal das Atemgerät beaufschlagt. Alternativ oder zusätzlich kann jedoch auch eine Reinigung im Umwälzbetrieb erfolgen, indem Reinigungsfluid mehrfach auf das Atemgerät aufgebracht wird. Derartige Umwälzbetriebe und Umwälzkreisläufe sind beispielsweise aus herkömmlichen Spülmaschinen oder Waschmaschinen bekannt.

Die Reinigungsvorrichtung weist weiterhin mindestens eine Druckbeaufschlagungsvorrichtung mit mindestens einem Druckanschluss auf. Unter einer Druckbeaufschlagungsvorrichtung ist allgemein eine Vorrichtung zu verstehen, welche eingerichtet ist, um ein Fluid, insbesondere ein Gas, mit einem Druck oberhalb des Normaldrucks bereitstellen zu können, beispielsweise einen Druck von mindestens 1,5 bar, vorzugsweise einen Druck von mindestens 2 bar. Unter einem Druckanschluss ist grundsätzlich ein beliebiger Anschluss der Druckbeaufschlagungsvorrichtung zu verstehen, über welchen das Fluid, insbesondere das Gas, der Druckbeaufschlagungsvorrichtung bereitstellbar ist. Insbesondere kann dieser Druckanschluss im Inneren der Reinigungskammer angeordnet sein und/oder vom Inneren der Reinigungskammer aus zugänglich sein. Auch eine Bereitstellung einer Mehrzahl von Druckanschlüssen ist denkbar. Der Druckanschluss soll mit mindestens einem gasführenden Element des Atemgeräts in der Reinigungskammer, vorzugsweise mit mehreren gasführenden Elementen mehrerer Atemgeräte in der Reinigungskammer, verbindbar sein. Unter einem gasführenden Element ist dabei allgemein ein Element zu verstehen, welches bei einer Benutzung des Atemgeräts durch einen menschlichen oder tierischen Benutzer mit einem Atemgas beaufschlagt wird oder auf andere Weise mit dem Atemgas in Kontakt kommen kann. Insbesondere kann es sich hierbei um einen Lungenautomaten und/oder einen Atemschlauch mit mindestens einem Ventil handeln. Insbesondere kann es sich bei dem gasführenden Element um ein Element handeln, welches mindestens einen Schlauch und/oder mindestens eine andere Art von Gasführungsvorrichtung mit einem Innenraum und/oder mindestens ein Ventil aufweist, welche in der Regel nicht mit Reinigungsfluid beaufschlagt werden dürfen. Beispielsweise kann es sich bei dem gasführenden Element um einen Bereich des Atemgeräts handeln, welcher im Betrieb mit einem Druck oberhalb des Normaldrucks beaufschlagt wird, beispielsweise einen gasführenden Bereich eines Atemgeräts oberhalb eines Normaldrucks, beispielsweise oberhalb von 1,5 bar, insbesondere oberhalb von 2 bar. Insbesondere kann es sich um einen atemgasführenden Bereich eines Lungenautomaten handeln, wobei es sich bei dem Lungenautomaten grundsätzlich um einen einstufigen oder auch einen mehrstufigen Lungenautomaten handeln kann. Dabei kann das gasführende Element beispielsweise ein Teil einer ersten Stufe und/oder einer zweiten Stufe und/oder gegebenenfalls weiterer Stufen des Lungenautomaten sein oder auch ein vollständiger Lungenautomat. Beispielsweise kann es sich bei dem gasführenden Element um einen Bereich zwischen einem Schlauchanschluss und einem Ventil eines Lungenautomaten handeln, oder das gasführende Element kann einen derartigen Bereich umfassen. Unter einer Verbindung zwischen dem Druckanschluss und dem gasführenden Element ist allgemein eine fluidische Verbindung, insbesondere eine Gasverbindung, zu verstehen, so dass das unter Druck stehende Fluid der Druckbeaufschlagungsvorrichtung in das gasführende Element des Atemgeräts, insbesondere in einen Innenraum des gasführenden Elements, überführt werden kann, vorzugsweise ohne dass bei dieser Verbindung ein Fluidverlust und/oder Druckverlust auftritt. Darüber hinaus kann optional die Verbindung zwischen dem Druckanschluss und dem gasführenden Element des Atemgeräts mindestens eine mechanische Verbindung umfassen, insbesondere eine formschlüssige und/oder kraftschlüssige Verbindung, so dass das Atemgerät fest mit dem Druckanschluss verbunden werden kann. Beispielsweise kann es sich hierbei um eine Schraubverbindung und/oder eine Klemmverbindung und/oder eine Spannverbindung handeln, wozu der Druckanschluss und/oder das Atemgerät jeweils mindestens ein mechanisches Verbindungselement aufweisen können. Insbesondere kann es sich hierbei um eine Steckverbindung in Form einer Schnellkupplung und/oder um ein Gewinde handeln, beispielsweise ein Kupplungsstück für einen Atemanschluss eines Lungenautomaten. Unter einer Schnellkupplung ist dabei allgemein eine gasdichte und/oder flüssigkeitsdichte Steckverbindung zwischen zwei Fluid-führenden Bauelementen zu verstehen, welche durch eine auf einfache und schnelle Weise herzustellende und zu lösende mechanische Fixierung, insbesondere ohne Verwendung eines Schraubverschlusses, mechanisch gesichert werden kann, beispielsweise durch mindestens einen Spannhaken und/oder einen Bajonettverschluss und/oder einen Schraubverschluss, beispielsweise eine Überwurfmutter. Die Verbindung kann insbesondere ohne Werkzeug herstellbar sein. Insbesondere kann der Druckanschluss eine Mehrzahl von fest oder austauschbar ausgestalteten Adaptern zur Verbindung mit unterschiedlichen Arten gasführender Elemente umfassen. Mittels dieser Adapter können eine Mehrzahl unterschiedlicher gasführender Elemente direkt oder indirekt mit dem Druckanschluss verbindbar sein, beispielsweise unterschiedliche Arten und/oder unterschiedliche Typen gasführender Elemente, beispielsweise unterschiedlicher Fabrikate oder Hersteller. Beispielsweise kann ein Adaptersatz mit einer Mehrzahl unterschiedlicher Kupplungsstücke (beispielsweise Kupplungsstücke einer Schnellkupplung) und/oder Gewinde zum Anschluss unterschiedlicher gasführender Elemente vorgesehen sein. Beispielsweise kann es sich hierbei um unterschiedliche Schnellkupplungssysteme und/oder Normgewinde handeln..Alternativ oder zusätzlich kann der mindestens eine Druckanschluss jedoch auch als fester Druckanschluss für einen bestimmten Typ von Verbindungselement bzw. für einen bestimmten Typ von gasführendem Element ausgestaltet sein. Besonders vorteilhaft ist es, wenn der Druckanschluss und/oder das Kupplungssystem so eingerichtet sind, dass einerseits kein Gas austreten kann wenn kein Gegenstück angeschlossen ist, und dass in diesem Fall auch kein anderes Medium, beispielsweise Reinigungsfluid, in das gasführende Element eintreten kann.

Die Druckbeaufschlagungsvorrichtung ist eingerichtet, um das gasführende Element während der Beaufschlagung mit dem Reinigungsfluid mit Druckgas zu beaufschlagen. Unter einem Druckgas ist dabei allgemein ein beliebiges Gas zu verstehen, welches einen Druck oberhalb des Normaldrucks, also einen Druck oberhalb von 1 bar, aufweist. Insbesondere kann es sich dabei um einen Druck oberhalb von 1,5 bar, insbesondere oberhalb von 2 bar, und besonders bevorzugt oberhalb von 3 bar handeln. Besonders bevorzugt ist die Druckbeaufschlagungsvorrichtung derart eingerichtet, dass die Druckbeaufschlagung mit dem Druckgas derart erfolgt, dass sämtliches Reinigungsfluid aus einem mit dem Druckgas beaufschlagten Innenraum des Atemgeräts ferngehalten wird. Bei dem Druckgas kann es sich beispielsweise um Druckluft oder ein anderes gasförmiges Medium mit einem Überdruck handeln, beispielsweise Stickstoff, Kohlendioxid oder ähnliches. Insbesondere kann ein Inertgas als Druckgas eingesetzt werden. Die Reinigungsvorrichtung kann insbesondere derart ausgestaltet sein, dass die Druckbeaufschlagung über die Druckbeaufschlagungsvorrichtung während mindestens eines Reinigungsvorgangs erfolgt, beispielsweise während mindestens eines Programmschritts eines einschrittigen oder mehrschrittigen Reinigungsprogramms. Insbesondere kann die Beaufschlagung mit dem Druckgas gleichzeitig zur Beaufschlagung des Atemgeräts mit dem Reinigungsfluid erfolgen. Der Druckanschluss kann dementsprechend insbesondere innerhalb der Reinigungskammer angeordnet sein, so dass die gleichzeitige Beaufschlagung mit Druckgas und Reinigungsfluid erfolgen kann.

Die Reinigungsvorrichtung kann auf verschiedene Weisen vorteilhaft weitergebildet werden. So kann, wie oben ausgeführt, der Druckanschluss mindestens ein formschlüssiges und/oder kraftschlüssiges Verbindungselement umfassen, um zusätzlich eine mechanische Verbindung mit dem Atemgerät herzustellen. Hierbei kann es sich insbesondere um ein Schnellkupplungssystem und/oder ein Gewinde handeln. Alternativ oder zusätzlich sind auch andere mechanische Verbindungselemente einsetzbar.

Die Reinigungsvorrichtung kann insbesondere als Einkammerspülmaschine ausgestaltet sein und/oder eine Einkammerspülmaschine umfassen. Unter einer Einkammerspülmaschine ist dabei eine Spülmaschine mit einer einzelnen Reinigungskammer zu verstehen, in welcher vorzugsweise mehrere Programmschritte eines mehrstufigen Reinigungsprogramms ausgeführt werden können. Die Beaufschlagung innerhalb einer Einkammerspülmaschine kann insbesondere durch eine Fluideinrichtung in Form einer oder mehrerer Düsen erfolgen, beispielsweise in Form einer oder mehrerer Sprühdüsen und/oder anderer Düsen, beispielsweise starren und/oder drehbaren und/oder schwenkbaren Düsenarmen. Die Einkammerspülmaschine kann insbesondere als so genannte Mehrkreis-Spülmaschine ausgestaltet sein. Dementsprechend kann die Einkammerspülmaschine beispielsweise mindestens einen separat von der Reinigungskammer ausgebildeten Fluidtank aufweisen, wobei in dem Fluidtank mindestens ein Reinigungsfluid unabhängig von einem in der Reinigungsvorrichtung momentan ablaufenden Reinigungsprozess konditionierbar ist, beispielsweise erwärmbar und/oder mit Zusätzen beaufschlagbar ist. Derartige Spülmaschinen mit einem Mehrkreisprinzip sind aus dem Bereich gewerblicher Geschirrspülmaschinen bekannt, bei welchen, unabhängig von der Reinigungskammer, in der Regel ein Tank für eine Konditionierung einer Nachspülflüssigkeit vorgesehen ist, beispielsweise ein Boiler und/oder ein Tank mit einem Durchlauferhitzer.

Die Bereitstellung des Druckgases kann durch die Reinigungsvorrichtung selbst erfolgen und/oder durch eine externe Vorrichtung So kann die Druckbeaufschlagungsvorrichtung insbesondere mindestens einen externen Druckanschluss zur Verbindung mit einer externen Druckquelle aufweisen. Bei dieser externen Druckquelle kann es sich beispielsweise um eine getrennt von der Reinigungsvorrichtung ausgebildete Druckgasflasche und/oder eine bauseitige Druckgasleitung, beispielsweise eine Pressluftleitung, handeln. Alternativ oder zusätzlich kann die Druckbeaufschlagungsvorrichtung auch mindestens eine in die Reinigungsvorrichtung integrierte Druckgasquelle aufweisen. Insbesondere kann mindestens eine integrierte Druckgasflasche vorgesehen sein und/oder mindestens ein integrierter Kompressor, zur Bereitstellung des Druckgases. Alternativ oder zusätzlich zur Bereitstellung eines einzelnen Druckgases können auch mehrere Druckgase gleichzeitig oder nacheinander bereitgestellt werden.

Die Reinigungsvorrichtung weist mindestens eine in die Reinigungskammer einbringbare Halterung zur Aufnahme des Atemgeräts auf. Diese Halterung kann fest in der Reinigungskammer installiert sein, kann jedoch auch beweglich ausgestaltet sein und/oder kann als herausnehmbare Halterung ausgestaltet werden, welche aus der Reinigungskammer herausgenommen werden kann, vorzugsweise ohne dass hierfür zusätzliches Werkzeug erforderlich wäre. Die Halterung ist eingerichtet, um das Atemgerät relativ zu der Fluideinrichtung auszurichten und vorzugsweise zu fixieren. Beispielsweise kann diese Fixierung derart erfolgen, dass das Atemgerät zwar an kritischen Stellen mit Reinigungsfluid beaufschlagt wird, wobei das Reinigungsfluid jedoch nach der Beaufschlagung vorzugsweise aus diesen kritischen Bereichen herausfließen kann, beispielsweise aus konkaven Wölbungen von Atemmasken. Die Halterung kann auch schwenkbar ausgestaltet sein, so dass während verschiedener Schritte eines Reinigungsverfahrens unterschiedliche Stellungen eingenommen werden können. Beispielsweise kann für eine Beaufschlagung mit dem Reinigungsfluid mindestens eine erste Stellung vorgesehen sein, und für ein Entleeren der Atemgeräte mindestens eine zweite Stellung. Die mindestens eine erste Stellung kann auch während des Programms wechseln, so dass eine Beaufschlagung aus mehreren Richtungen erfolgen kann, ohne dass hierfür die Fluideinrichtung, beispielsweise die Düsen, in ihrer Stellung und/oder Ausrichtung verändert werden müssten, was jedoch gleichwohl erfolgen kann. Ein Entleeren kann beispielsweise durch ein Verkippen und/oder Verdrehen der Halterung erfolgen, so dass beispielsweise Atemmasken und/oder Innenbereiche von Schläuchen entleert werden können. Alternativ oder zusätzlich zu einer Halterung zur Fixierung des Atemgeräts ist jedoch grundsätzlich eine nicht-ausgerichtete und/oder nicht-fixierte Aufnahme der Atemgeräte in der Reinigungskammer möglich.

Allgemein kann die Reinigungsvorrichtung dementsprechend eine modifizierte Geschirrspülmaschine und/oder eine modifizierte Wäschewaschmaschine sein oder eine derartige Geschirrspülmaschine oder Wäschewaschmaschine im modifizierten Zustand umfassen. Die Reinigungsvorrichtung ist derart eingerichtet, dass die Beaufschlagung mit Reinigungsfluid durch Bestrahlen, Besprühen oder Betropfen erfolgt. Das Atemgerät taucht während der Beaufschlagung nicht in das Reinigungsfluid ein, so dass das Reinigungsfluid unmittelbar bei oder unmittelbar nach dem Auftreffen auf das Atemgerät wieder von diesem abfließen und/oder abtropfen kann. Auf diese Weise kann verhindert werden, dass durch eine längere Einwirkung oder sogar durch eine Beaufschlagung des Atemgeräts mit Reinigungsfluid bei Überdruck das Reinigungsfluid in das gasführende Element eindringt, und/oder es kann verhindert werden, dass Rückstände des Reinigungsfluids in dem Atemgerät verbleiben. Die Reinigungsvorrichtung ist als Spülmaschine ausgestaltet, bei welcher das Atemgerät in der Halterung aufgenommen ist und aus mindestens einer Düse von oberhalb und unterhalb der Halterung mit Reinigungsfluid beaufschlagt wird, wobei nach der Beaufschlagung das Reinigungsfluid unmittelbar von dem Atemgerät in einen Waschtank abfließen und/oder abtropfen kann.

Die Halterung kann insbesondere derart ausgestaltet sein, dass diese mehrere unterschiedliche Aufnahmen aufweist, die eingerichtet sind, um unterschiedliche Arten von Atemgerät und/oder unterschiedliche Bestandteile des Atemgeräts aufzunehmen. Insbesondere kann die Halterung mehrere Fächer und/oder Aufnahmen umfassen, in denen unterschiedliche Bestandteile eines Atemgeräts aufgenommen werden können und ggf. fixiert und/oder räumlich ausgerichtet werden können, wobei die Bestandteile dennoch einander zugeordnet bleiben, so dass nach der Reinigung die Bestandteile ohne Untermischung wieder einander zugeordnet und zusammengefügt werden können. So kann die Halterung beispielsweise mindestens zwei Halterungsgruppen mit jeweils einer Mehrzahl von Aufnahmen umfassen, wobei jeweils eine Halterungsgruppe einem Atemgerät zugeordnet ist und wobei jeweils eine Halterungsgruppe mehrere unterschiedliche Arten von Aufnahmen zur Aufnahme unterschiedlicher Bestandteile eines Atemgeräts umfasst.

Das mindestens eine, oben beschriebene formschlüssige und/oder kraftschlüssige Verbindungselement zur mechanischen Verbindung mit dem Atemgerät, welches optional vorgesehen werden kann, kann insbesondere ganz oder teilweise in der Halterung integriert sein. Auch der mindestens eine Druckanschluss kann ganz oder teilweise in die Halterung integriert sein. So kann beispielsweise der Druckanschluss einen oder mehrere Anschlussstutzen zur Verbindung mit dem mindestens einen Atemgerät umfassen, wobei der mindestens eine Anschlussstutzen beispielsweise über einen oder mehrere zentrale Beaufschlagungsstutzen mit weiteren Elementen der Druckbeaufschlagungsvorrichtung, insbesondere einer integrierten und/oder externen Druckgasquelle, verbunden werden kann. Beispielsweise kann eine Schlauchverbindung und/oder eine Rohrverbindung zu der Halterung hergestellt werden, welche beispielsweise beim Herausnehmen der Halterung aus der Reinigungskammer lösbar ausgestaltet sein kann. Alternativ oder zusätzlich zu einer Integration des Druckanschlusses in die Halterung kann jedoch auch grundsätzlich eine andere Art der Ausgestaltung des Druckanschlusses erfolgen, beispielsweise eine einfache Schlauchverbindung zu beliebig in der Reinigungskammer angeordneten Atemschutzmasken. Die Halterung kann insbesondere eine lösbare Druckgasverbindung zur restlichen Reinigungsvorrichtung aufweisen, insbesondere zu einem oder mehreren optionalen sonstigen Bestandteilen der Druckbeaufschlagungsvorrichtung, welche nicht in die Halterung integriert sind. Wie oben ausgeführt, können beispielsweise die Halterung und/oder der Druckanschluss und/oder sonstige Bestandteile der Druckbeaufschlagungsvorrichtung mindestens einen Druckverteiler umfassen, mittels dessen, insbesondere von der mindestens einen optionalen lösbaren Druckgasverbindung als zentralem Druckanschluss und als zentraler Verbindung zur restlichen Reinigungsvorrichtung, Druckgas auf mehrere Atemgeräte und/oder mehrere gasführende Elemente verteilt werden kann. Der Druckverteiler kann beispielsweise mehrere Anschlüsse für gasführende Elemente umfassen, beispielsweise in einer linearen und/oder einer sternförmigen Anordnung.

Wie oben ausgeführt, kann die Reinigungsvorrichtung insbesondere als Programmautomat eingerichtet sein, um ein Reinigungsprogramm mit einem oder mehreren Programmschritten durchzuführen. Insbesondere kann die Reinigungsvorrichtung eingerichtet sein, um ein Reinigungsprogramm mit mindestens zwei unterschiedlichen Programmschritten durchzuführen. Beispielsweise kann bei unterschiedlichen Programmschritten eine Beaufschlagung mit unterschiedlichen Arten von Reinigungsfluiden erfolgen, beispielsweise mindestens ein Reinigungsschritt, in welchem eine Beaufschlagung mit einer Reinigerlösung erfolgt, und mindestens ein weiterer Programmschritt, insbesondere ein Nachspülschritt, in welchem eine Beaufschlagung mit einem Nachspülfluid erfolgt. Optional kann gegebenenfalls auch mindestens ein Programmschritt vorgesehen sein, welcher als Trocknungsschritt ausgestaltet ist, wobei die Trocknung gegebenenfalls passiv erfolgen kann, beispielsweise durch einfaches Abtropfen, oder, alternativ, bei welchem die Trocknung auch aktiv unterstützt werden kann, beispielsweise durch automatisiertes Ausblasen der Masken mit Druckluft und/oder durch Vorsehen einer Wärmequelle wie Warmluft, Mikrowellen und/oder mindestens einer ähnlichen Wärme- oder Trocknungsquelle.

Weitere mögliche Ausgestaltungen betreffen die Fluideinrichtung. Die Fluideinrichtung weist mindestens eine Düse auf. Insbesondere kann es sich dabei um eine Düse handeln, welche ausgewählt ist aus einer Sprühdüse, einer Spüldüse, einem Sprüharm, insbesondere einem schwenkbaren Sprüharm, einer im Umwälzbetrieb betreibbaren Düse. Auch eine Kombination der genannten und/oder anderer Arten von Düsen ist denkbar. Alternativ oder zusätzlich kann die Fluideinrichtung jedoch auch beispielsweise eine einfache Öffnung zum Einlass von Reinigungsfluid in die Reinigungskammer umfassen.

Das Reinigungsfluid kann insbesondere ein wässriges Reinigungsfluid umfassen, also Wasser oder Wasser mit Zusatz von einem oder mehreren Zusätzen, welche in gelöster Form, in Emulsionsform oder auch in Suspensionsform vorliegen können. Alternativ oder zusätzlich kann das Reinigungsfluid auch mindestens eine Reinigerlösung umfassen. Unter einer Reinigerlösung ist dabei allgemein eine Lösung mindestens eines Tensids in mindestens einem Lösungsmittel, beispielsweise ebenfalls Wasser, zu verstehen. Beispielsweise kann es sich hierbei um eine kommerzielle Reinigerlösung handeln, welche auch für Geschirrspülmaschinen eingesetzt werden kann und/oder welche in Waschmaschinen eingesetzt werden kann. Auch spezielle Reinigerlösungen können jedoch grundsätzlich entwickelt und/oder eingesetzt werden. Wiederum alternativ oder zusätzlich kann das Reinigungsfluid auch ein Reinigungsfluid mit mindestens einem Klarspüler umfassen, also einem Zusatz, welcher ein Abtrocknen des Atemgeräts erleichtert und/oder ein Abperlen von Flüssigkeit von mindestens einer Oberfläche des Atemgeräts erleichtert. Derartige Klarspüler, welche ebenfalls ein oder mehrere Tenside umfassen können, sind aus dem Bereich der Geschirrspültechnik grundsätzlich bekannt. Wiederum alternativ oder zusätzlich kann das Reinigungsfluid auch mindestens ein Nachspülfluid umfassen, welches grundsätzlich beispielsweise Wasser sein kann und/oder Wasser mit einem oder mehreren Zusätzen. Wiederum alternativ oder zusätzlich kann das Reinigungsfluid auch ein Reinigungsfluid mit mindestens einem Desinfektionsmittel umfassen, wobei unter einem Desinfektionsmittel grundsätzlich eine beliebige Substanz verstanden werden kann, welche eine keimabtötende Wirkung aufweist. Derartige Desinfektionsmittel sind aus dem Stand der Technik ebenfalls grundsätzlich bekannt. Wiederum alternativ oder zusätzlich kann das Reinigungsfluid auch demineralisiertes Wasser umfassen. Wie unten noch näher ausgeführt wird, kann das demineralisierte Wasser beispielsweise in einer entsprechenden Demineralisierungsvorrichtung der Reinigungsvorrichtung bereitgestellt werden, beispielsweise einem Ionentauscher.

Alternativ oder zusätzlich kann die Demineralisierungsvorrichtung jedoch auch beispielsweise eine Umkehrosmosevorrichtung umfassen. Wiederum alternativ oder zusätzlich kann das Reinigungsfluid auch ein erwärmtes Reinigungsfluid umfassen. Insbesondere zum Nachspülen können sich derartige erwärmte Reinigungsfluide eignen, wobei unter erwärmten Reinigungsfluiden grundsätzlich Reinigungsfluide zu verstehen sind, welche eine Temperatur von mindestens 25 °C aufweisen. Besonders bevorzugt sind Temperaturen von 30 °C bis 70 °C und insbesondere 60 °C. Diese Temperaturen haben sich insbesondere für die Reinigung von flexiblen Elastomerwerkstoffen, wie sie für Atemgeräte häufig eingesetzt werden, als geeignet erwiesen.

Wie oben dargestellt, ist für die Reinigung von Atemgeräten die Bereitstellung qualitativ hochwertiger Reinigungsfluide von besonderer Bedeutung. Auf diese Weise kann beispielsweise eine mikroskopische oder auch makroskopische Verunreinigung der Atemgeräte vermieden werden. Durch mikroskopische Verunreinigungen wie beispielsweise mineralische Verunreinigungen entstehen beispielsweise Kalkablagerungen auf Sichtflächen wie beispielsweise Sichtscheiben der Atemgeräte. Weiterhin können mikrobielle Verunreinigungen zu einer Kontamination der Atemgeräte führen. Besonders bevorzugt ist es daher, wenn die Reinigungsvorrichtung mindestens eine Umkehrosmosevorrichtung zur Bereitstellung von Wasser aufweist, insbesondere von demineralisiertem Wasser. Unter einer Umkehrosmosevorrichtung ist allgemein eine Vorrichtung zu verstehen, welche ein Fluid mit hohem Reinheitsgrad bereitstellen kann, unter Verwendung des Prinzips der Umkehrosmose. Beispielsweise kann die Umkehrosmosevorrichtung eine oder mehrere Osmosemembranen aufweisen, durch welche unter hohem Druck saubere Bestandteile des Wassers gepresst werden können, so dass ein aufgereinigtes Permeat entsteht, wohingegen auf einer Konzentratseite Verunreinigungen zurückbleiben. Beispielsweise können hierbei Drücke von oberhalb von 2 bar, insbesondere oberhalb von 5 bar, eingesetzt werden.

Wie oben dargestellt, können zum Zwecke der vorliegenden Erfindung insbesondere kommerziell erhältliche Wäschewaschmaschinen und/oder Geschirrspülmaschinen umgestaltet werden, beispielsweise gewerbliche Wäschewaschmaschinen und/oder gewerbliche Geschirrspülmaschinen. Diese Umgestaltung kann derart erfolgen, dass die Wäschewaschmaschinen bzw. Geschirrspülmaschinen mit einer Druckbeaufschlagungsvorrichtung gemäß den oben beschriebenen Merkmalen ausgestattet werden. Die Reinigungsvorrichtung kann beispielsweise als Frontladerspülmaschine oder als Haubenspülmaschine ausgestaltet sein. Unter einer Frontladerspülmaschine ist dabei eine Spülmaschine mit einer Reinigungskammer zu verstehen, welche durch eine Klappe und/oder einen Schieber durch einen vor der Spülmaschine angeordneten Benutzer geöffnet werden kann. Unter einer Haubenspülmaschine ist dabei eine Spülmaschine zu verstehen, deren Reinigungskammer eine aufschwenkbare und/oder auffahrbare Haube umfasst, welche von sonstigen Bestandteilen der Reinigungskammer, beispielsweise einer Basis, getrennt werden kann. Derartige Haubenspülmaschinen sind grundsätzlich aus dem Bereich der gewerblichen Geschirrspültechnik bekannt. Die Reinigungskammer kann insbesondere starr ausgestaltet sein. Die Reinigungskammer kann allgemein während des Reinigungsvorgangs verriegelbar ausgestaltet sein, insbesondere indem eine automatische Verriegelung vorgesehen ist, welche vorzugsweise während eines Programms ausgelöst wird und nach einem Programm automatisch wieder gelöst werden kann.

In einem weiteren Aspekt wird eine Halterung zum Einsatz in einer Reinigungsvorrichtung gemäß einer oder mehreren der oben beschriebenen Ausgestaltungen offenbart. Dementsprechend kann für mögliche Ausgestaltungen der Halterung auf die obige Beschreibung verwiesen werden und dort insbesondere auf die die Halterung betreffenden Merkmale. Die Halterung soll in die Reinigungskammer einbringbar sein. Diese Einbringung kann permanent oder auch reversibel erfolgen, beispielsweise indem die Halterung mindestens ein Verbindungselement zur Verbindung mit der Reinigungskammer aufweist. Beispielsweise kann die Halterung eine oder mehrere Rollen und/oder Räder und/oder sonstige Arten von Verbindungselementen umfassen, welche in eine oder mehrere Schienen innerhalb der Reinigungskammer einschiebbar sind. Auch andere Ausgestaltungen der Verbindungselemente sind möglich.

Die Halterung ist eingerichtet, um mindestens ein Atemgerät aufzunehmen. Vorzugsweise ist die Halterung auch eingerichtet, um das Atemgerät relativ zu der Fluideinrichtung auszurichten. Die Ausrichtung kann dabei vollständig oder teilweise erfolgen, beispielsweise indem lediglich ein Teil des Atemgeräts relativ zu einer oder mehreren Düsen ausgerichtet wird, so dass besonders kritische Teile routinemäßig und zuverlässig mit Reinigungsfluid beaufschlagt werden können. Die Ausrichtung kann dabei starr erfolgen oder auch, wie oben ausgeführt, bewegbar, beispielsweise schwenkbar und/oder drehbar. Beispielsweise kann die Reinigungsvorrichtung derart ausgestaltet sein, dass in unterschiedlichen Programmschritten unterschiedliche Positionen und/oder unterschiedliche Ausrichtungen des Atemgeräts vorgesehen sind. Die Halterung weist mindestens einen integrierten Druckanschluss auf, wobei der Druckanschluss als Bestandteil der Druckbeaufschlagungsvorrichtung wirken kann und eingerichtet ist, um mit mindestens einem gasführenden Element des Atemgeräts verbunden zu werden. Wie oben ausgeführt, kann zusätzlich mindestens ein mechanisches Verbindungselement vorgesehen sein, beispielsweise ein formschlüssiges und/oder kraftschlüssiges Verbindungselement, welches eine mechanische Verbindung zwischen der Halterung und dem Atemgerät herstellen kann. Insbesondere kann mindestens eine Schnellkupplung und/oder mindestens ein Gewinde innerhalb der Halterung vorgesehen sein. Für weitere mögliche Ausgestaltungen der Halterung, welche beispielsweise als Zubehörteil und/oder als Austauschteil ausgestaltet sein kann, kann auf die obige Beschreibung verwiesen werden. Insbesondere kann eine Bestückung der Halterung mit einem oder mehreren Atemgeräten außerhalb der Reinigungskammer erfolgen, bevor die bestückte Halterung in die Reinigungskammer eingebracht werden kann. Verschiedene Ausgestaltungen sind möglich.

Die oben beschriebene Reinigungsvorrichtung kann auf verschiedene Weisen vorteilhaft verwendet werden. Insbesondere wird dementsprechend erfindungsgemäß eine Verwendung der Reinigungsvorrichtung in einer oder mehreren der oben dargestellten Ausgestaltungen zur Reinigung mindestens eines Atemgeräts vorgeschlagen, wobei das Atemgerät insbesondere ausgewählt sein kann aus: einem Lungenautomaten oder einer Atemmaske für Taucher; einem Lungenautomaten oder einer Atemschutzmaske für Rettungskräfte; einem Pressluftatmer; einem Lungenautomaten oder einer Atemschutzmaske für Polizei oder Streitkräfte oder andere Sicherheitskräfte; einem Lungenautomaten oder einer Atemmaske für medizinische Zwecke. Auch andere Verwendungen sind jedoch grundsätzlich möglich.

In einem weiteren Aspekt der vorliegenden Erfindung wird ein Verfahren zur Reinigung eines Atemgeräts vorgeschlagen. Insbesondere kann es sich hierbei um einen Lungenautomaten und/oder eine Atemmaske, beispielsweise eine Atemschutzmaske, handeln. Insbesondere kann das Atemgerät, wie oben dargestellt, mindestens einen Bereich mit mindestens einem Ventil umfassen, durch welches ein Innenraum, beispielsweise ein gasführender Innenraum eines gasführenden Elements, von einem Außenraum getrennt werden kann, wobei das Verfahren insbesondere derart durchgeführt werden kann, dass der Innenraum nicht mit Reinigungsfluid beaufschlagt wird. Insbesondere kann es sich bei diesem Ventil um mindestens einen Kipphebel eines Lungenautomaten handeln. Auch andere Ausgestaltungen sind jedoch grundsätzlich möglich. Das Verfahren wird unter Verwendung einer Reinigungsvorrichtung gemäß einer oder mehreren der oben beschreiben Ausgestaltungen durchgeführt, so dass bezüglich optionaler Ausgestaltungen auf die obige Beschreibung verwiesen werden kann.

Bei dem erfindungsgemäßen Verfahren wird das Atemgerät mit mindestens einem Reinigungsfluid beaufschlagt. Während der Beaufschlagung mit dem Reinigungsfluid wird mindestens ein gasführendes Element des Atemgeräts mit einem Druckgas beaufschlagt. Die Beaufschlagung mit dem Druckgas kann insbesondere derart erfolgen, beispielsweise durch eine geeignete Wahl des Durchsatzes und/oder des Drucks des Druckgases, dass im Wesentlichen kein Reinigungsfluid ins Innere des gasführenden Elements eindringen kann. Dabei kann eine vollständige Verhinderung eines Eindringens erfolgen. Alternativ kann jedoch auch eine Tolerierung geringer Mengen, beispielsweise geringer Mengen im Mikro- oder Nanoliterbereich, erfolgen.

Die oben beschriebene Reinigungsvorrichtung, die oben beschriebene Halterung, die Verwendung und das vorgeschlagene Verfahren weisen gegenüber bekannten Verfahren und Vorrichtungen eine Vielzahl von Vorteilen auf. So kann erfindungsgemäß insbesondere eine kurze Reinigungszeit, beispielsweise im Rahmen eines Reinigungsprogramms, bei gleichzeitig hoher Reinigungsleistung und geringem Verbrauch von Energie und Wasser realisiert werden. Diese Realisierung kann insbesondere mittels einer Waschmaschine oder einer Spülmaschine erfolgen, die im so genannten Zweikreisprinzip arbeiten, ähnlich den Ausführungen, wie sie beim gewerblichen Spülen von Geschirr bekannt sind, beispielsweise in Form von Einkammer-Programmautomaten. Es sind dabei beispielsweise Bauformen als Frontlader- oder Hauben-Modelle denkbar.

Die Verwendung einer Halterung, insbesondere einer herausnehmbaren Halterung, für die Atemgeräte ermöglicht beispielsweise die Befestigung einer oder mehrerer Atemgeräte, beispielsweise Atemschutzmasken, in einer Weise, dass einerseits alle Oberflächen erreicht werden und andererseits das Reinigungsfluid gut abläuft. Beispielsweise kann eine Positionierung relativ zu den Sprühstrahlen und/oder anderen Fluideinrichtungen erfolgen, so dass alle kritischen Oberflächen von dem Reinigungsfluid gut erreicht werden. Andererseits kann durch eine geeignete Ausrichtung erreicht werden, dass sich keine Ansammlungen von Reinigungsfluidresten im Inneren der Atemgeräte, beispielsweise im Inneren von Masken, bilden können. Beispielsweise kann die Halterung zur Aufnahme von zwei, drei, vier, fünf oder mehr Atemgeräten, beispielsweise zur Aufnahme einer derartigen Anzahl an Atemmasken und/oder Lungenautomaten, ausgebildet sein. Die Halterung, wobei auch mehrere Halterungen vorgesehen sein können, kann insbesondere derart ausgestaltet sein, dass Zubehör der Atemgeräte jeweils einem Atemgerät zugeordnet bleiben kann. So kann beispielsweise Zubehör einer einzelnen Atemmaske zugeordnet sein, indem beispielsweise die Halterung ein korbartiges Behältnis umfasst, in welchem das Zubehör aufgenommen sein kann. Auf diese Weise kann auch während des Reinigungsvorgangs die Zuordnung erhalten bleiben.

Die Reinigungsvorrichtung und insbesondere die Halterung kann insbesondere mit einer oder mehreren Druckanschlüssen für das Atemgerät ausgestattet sein, beispielsweise für einen oder mehrere Lungenautomaten, wobei mittels des mindestens einen Druckanschlusses unter Überdruck stehendes Gas, z.B. Druckluft, geführt werden kann. Der oder die Lungenautomaten können beim Beladen der Reinigungsvorrichtung mit ihrem gasführenden Bereich an diesen mindestens einen Druckanschluss angekoppelt werden. Durch den Kopplungsvorgang kann der gesamte gasführende Bereich oder ein Teil desselben für das mindestens eine Atemgerät, beispielsweise den Lungenautomaten, vom Gas durchflutet werden, das unter höherem Druck als der Umgebungsdruck stehen kann. So wird zuverlässig verhindert, dass während des Reinigungsvorgangs der restlichen Oberflächen Reinigungsfluid in den mindestens einen gasführenden Bereich eindringt.

Die Zufuhr des Druckgases für die Druckbeaufschlagungsvorrichtung kann auf verschiedene Weisen erfolgen. So kann von einem bauseitigen Druckgas-Anschluss eine Verbindung zur Reinigungsvorrichtung geschaffen sein. Alternativ oder zusätzlich kann bei oder innerhalb der Reinigungsvorrichtung ein Druckgasbehälter angebracht sein, der nach seiner Entleerung beispielsweise durch einen vollen Behälter ausgetauscht werden kann. Wiederum alternativ oder zusätzlich kann in der Reinigungsvorrichtung auch mindestens ein Kompressor vorgesehen sein, der beispielsweise direkt vor Ort Druckgas, insbesondere Druckluft, erzeugen kann.

Eine bestimmungsgemäße Verwendung der Reinigungsvorrichtung kann beispielsweise derart ausgestaltet sein, dass zunächst das mindestens eine Atemgerät, beispielsweise Atemschutzmasken, Zubehör und Lungenautomaten, vorschriftsmäßig an der Halterung befestigt werden. Anschließend kann optional die gesamte Halterung in die Reinigungskammer, beispielsweise den Spülraum der Spülmaschine, eingebracht werden. Optional kann vor, während oder nach dem Einbringen der Halterung eine Druckgasverbindung mit der Halterung hergestellt werden. Die Reinigungskammer kann dann verschlossen werden, und ein Reinigungsprogramm kann gestartet werden. So kann das Reinigungsprogramm beispielsweise derart ausgestaltet werden, dass in einem ersten Schritt das Atemgerät mit einem Reinigungsfluid, beispielsweise einer Reinigungsflüssigkeit, abgespült und/oder für einen gewissen Zeitraum umspült wird. Das Reinigungsfluid kann beispielsweise auf wässriger Basis ausgestaltet sein und kann mit einem oder mehreren Zusätzen versetzt sein. Diese Zusätze können vergleichbar sein mit Reinigungsmitteln, die bei der manuellen Reinigung von Atemschutzmasken derzeit zum Einsatz kommen. Die Zusätze können auf die Werkstoffe, aus denen die Atemschutzmasken bestehen, abgestimmt sein. Weiterhin kann das Reinigungsfluid eine oder mehrere Komponenten enthalten, die eine Desinfektion auf chemischer Basis bewirken, also eine oder mehrere Desinfektionsmittel. Das Reinigungsfluid kann erwärmt sein, beispielsweise auf 60 °C. Die Temperatur kann insbesondere abgestimmt werden auf die Erfordernisse der Werkstoffe, aus denen das Atemgerät zusammengesetzt ist, beispielsweise auf die Erfordernisse der Werkstoffe der Masken, und/oder kann beispielsweise aus Anforderungen der verwendeten Reinigungsmittel und/oder Desinfektionsmittel resultieren. In einem weiteren Programmschritt kann optional das mindestens eine Atemgerät mit einem Nachspülfluid, beispielsweise Frischwasser, nachgespült werden. Das Nachspülfluid kann beispielsweise ebenfalls erwärmt und/oder mit Klarspülmittel versetzt sein, welches das Ablaufen des Fluids und das Trocknen begünstigen kann. Besondere Vorteile für den Nachspüleffekt bringt es, wenn das Frischwasser entmineralisiert wird, beispielsweise unter Verwendung der oben beschriebenen Osmoseanlage. Optional kann die Reinigung auch in mehrere Schritte aufgeteilt sein, oder es können mehrere Nachspülschritte erfolgen. Die Dauer der einzelnen Programmschritte kann ebenfalls auf die Erfordernisse angepasst werden, beispielsweise Erfordernisse der eingesetzten Reinigungsmittel und/oder Desinfektionsmittel und/oder der Art der Atemgeräte und/oder einem Kontaminations- bzw. Verschmutzungsgrad. Zur erfolgreichen Durchführung einer chemothermischen Desinfektion sind beispielsweise Programmlaufzeiten von 5 Minuten bekannt. Am Ende eines Reinigungsprogramms kann dann optional die Halterung mit dem mindestens einen gereinigten und optional hygienisierten Atemgerät aus der Reinigungskammer, beispielsweise dem Spülraum, entnommen werden. Nach einer kurzen Wartezeit, welche innerhalb oder außerhalb der Reinigungskammer verbracht werden kann, sind in der Regel alle Flüssigkeitsreste von dem Atemgerät abgetropft bzw. abgetrocknet. Das Atemgerät kann dann von der Halterung abgenommen werden, und weitere Schritte der Aufbereitung können sich anschließen. Optional kann die Reinigungskammer während des Reinigungsprogramms manuell oder automatisch durch eine Maschinensteuerung verriegelt werden und am Ende eines ordnungsgemäßen Programmablaufs zur Entnahme des Atemgeräts freigegeben werden.

Die beschriebene Reinigungsvorrichtung und das vorgeschlagene Verfahren können auch an anderen Stellen eingesetzt werden, an denen Atemgeräte, beispielsweise Atemmasken und besonders Lungenautomaten, zum Einsatz kommen. Beispielsweise wurde oben bereits der Katastrophenschutz erwähnt, sowie Streitkräfte, Tauchsport sowie bestimmte medizinische Bereiche. In diesen und in anderen Bereichen machen sich die Vorteile der vorgeschlagenen Reinigungsvorrichtung und des vorgeschlagenen Verfahrens besonders günstig bemerkbar. Diese Vorteile bestehen insbesondere in einem geringen Zeitaufwand für die Reinigung des Atemgeräts und dessen Zubehör. Während ein automatisches Reinigungsprogramm ablaufen kann, kann eine Bedienperson weitere Arbeitsgänge im Rahmen einer Aufbereitung des Atemgeräts, beispielsweise der Maskenaufbereitung, ausführen, so dass sich ein rationeller Arbeitsablauf ergeben kann. Durch das gezielte Aufnehmen des Atemgeräts, beispielsweise der Masken, und das dadurch bewirkte gute Ablaufen von Reinigungsfluid kann die Zeit für den nachfolgend in der Regel vorgeschriebenen Trocknungsvorgang drastisch verringert werden. Das vorgeschlagene Verfahren und die vorgeschlagene Reinigungsvorrichtung ermöglichen somit eine rationelle und dennoch sichere Möglichkeit der Reinigung von Atemgerät, einschließlich eines oder mehrerer Lungenautomaten. Der Reinigungsprozess kann dabei standardisiert und überwacht werden, wodurch insbesondere eine gleichbleibende, hohe Reinigungsgüte und Hygiene gewährleistet werden kann. Für die Benutzer des Atemgeräts ergibt sich hierdurch ein höheres Vertrauen zu ihrer persönlichen Schutzausrüstung. Gleichzeitig kann durch ein standardisiertes Verfahren ein geringerer Ressourcenverbrauch als bei herkömmlichen Verfahren, insbesondere als bei manuellen Reinigungsverfahren, gewährleistet werden.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigen:
- Figur 1: eine Schnittdarstellung eines Ausführungsbeispiels einer erfindungsgemäßen Reinigungsvorrichtung; und
- Figur 2: eine Detaildarstellung einer bestückten Halterung der Reinigungsvorrichtung gemäß Figur 1.

### Ausführungsbeispiele

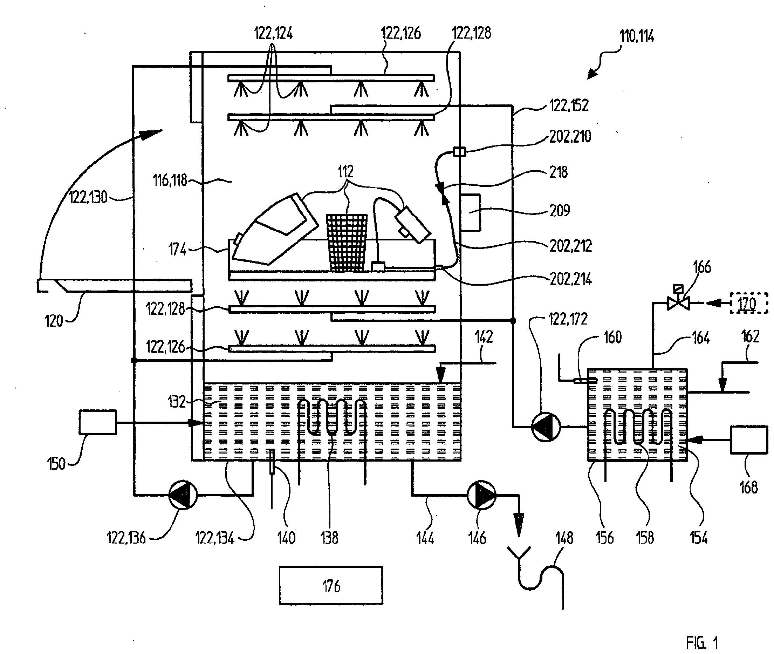

In Figur 1 ist ein Ausführungsbeispiel einer erfindungsgemäßen Reinigungsvorrichtung 110 dargestellt, welche zur Reinigung eines oder mehrerer Atemgeräte 112 eingerichtet ist. Die Reinigungsvorrichtung 110 ist in Figur 1 in einer Schnittdarstellung schematisch gezeigt. Die Reinigungsvorrichtung 110 kann beispielsweise als Spülmaschine 114 ausgestaltet sein. Die Reinigungsvorrichtung 110 umfasst eine Reinigungskammer 116, beispielsweise eine Spülkammer 118. Diese Reinigungskammer 116 kann beispielsweise durch eine Tür 120, beispielsweise eine Schwenktür, eine Schiebetür oder eine Klappe, und/oder durch eine andere Öffnungsvorrichtung geöffnet werden. Alternativ oder zusätzlich kann die Reinigungskammer 116 auch als durch eine Haube abgedeckte Reinigungskammer ausgestaltet sein. Auch andere Ausgestaltungen sind möglich. In dem in Figur 1 dargestellten Ausführungsbeispiel ist die Spülmaschine 114 beispielsweise als FrontladerSpülmaschine ausgestaltet. Auch andere Ausgestaltungen sind jedoch möglich.

Die Reinigungsvorrichtung 110 weist mindestens eine Fluideinrichtung 122 zur Beaufschlagung der in der Reinigungskammer 116 aufgenommenen Atemgeräte 112 mit einem oder mehreren Reinigungsfluiden auf. Die Fluideinrichtung 122 umfasst eine oder mehrere Düsen 124 umfassen, welche oberhalb und unterhalb der Atemgeräte 112 und optional an anderen Orten innerhalb der Reinigungskammer 116 angeordnet sind, beispielsweise an einer oder mehreren Seitenwänden. Beispielsweise kann die Fluideinrichtung 122, wie in dem dargestellten Ausführungsbeispiel gemäß Figur 1, ein Waschdüsensystem 126 umfassen, beispielsweise mit einem oder mehreren unterhalb und/oder oberhalb der Atemgeräte 112 aufgenommenen, vorzugsweise drehbar und/oder schwenkbar gelagerten Düsenarmen mit mehreren Düsen 124. Alternativ oder zusätzlich kann die Fluideinrichtung 122 auch ein Nachspüldüsensystem 128 umfassen, beispielsweise mit einem oder mehreren Nachspüldüsenarmen, welche vorzugsweise wiederum drehbar und/oder schwenkbar gelagert sind, welche beispielsweise wiederum oberhalb und/oder unterhalb der Atemgeräte 112 angeordnet sein können. Auch andere Anordnungen und/oder Ausgestaltungen sind jedoch grundsätzlich möglich.

Die Fluideinrichtung 122 kann darüber hinaus ein oder mehrere weitere Elemente umfassen, wie beispielsweise eine oder mehrere Rohrleitungen, eine oder mehrere Pumpen und/oder ein oder mehrere Tanks. So ist beispielsweise in dem dargestellten Ausführungsbeispiel mindestens ein Waschleitungssystem 130 vorgesehen, zur Beaufschlagung des Waschdüsensystems 126 mit Reinigungsfluid 132, beispielsweise Reinigerlösung, aus einem oder mehreren Waschtanks 134. Beispielsweise kann ein Waschtank 134 im Bodenbereich der Reinigungskammer 116 vorgesehen sein und/oder auf andere Weise mit der Reinigungskammer 116 verbunden sein, so dass Reinigungsfluid 132 nach Beaufschlagung der Atemgeräte 112 wieder zurück in den Waschtank 134 fließen und/oder tropfen kann. Zur Beaufschlagung des Waschdüsensystems 126 mit dem Reinigungsfluid 132 aus dem Waschtank 134 kann die Fluideinrichtung 122 weiterhin eine oder mehrere Umwälzpumpen 136 aufweisen. Weiterhin können ein oder mehrere Heizelemente 138 vorgesehen sein, um das Reinigungsfluid 132 des Waschtanks 134 und/oder anderer Tanks zu erwärmen, beispielsweise in Form einer Waschtankheizung innerhalb des Waschtanks 134. Zur Kontrolle der Aufheizung des Reinigungsfluids 132 können ein oder mehrere Temperatursensoren 140 vorgesehen sein, beispielsweise innerhalb des Waschtanks 134. Weiterhin können ein oder mehrere Niveausensoren 142 vorgesehen sein, beispielsweise ein Niveausensor 142 als Niveausensor des Waschtanks 134. Der Waschtank 134 kann beispielsweise über eine Ablaufleitung 144 und optional über eine Ablaufpumpe 146 in einen Ablauf 148 entleerbar sein. Optional können auch ein oder mehrere, in Figur 1 nicht dargestellte, Zuläufe zu dem Waschtank 134 vorgesehen sein, um diesen mit Reinigungsfluid 132 zu befüllen. Alternativ oder zusätzlich kann eine Befüllung jedoch auch über das nachfolgend noch näher beschriebene Nachspüldüsensystem 128 erfolgen. Weiterhin kann ein Dosiersystem 150 vorgesehen sein, beispielsweise mindestens ein Dosiersystem, um ein oder mehrere Zusätze in das Reinigungsfluid 132 des Waschtanks 134 einzubringen, beispielsweise ein Reinigerkonzentrat, ein Klarspülmittel, ein Desinfektionsmittel oder Kombinationen der genannten und/oder anderer Zusätze.

Das Reinigungsfluid 132 kann insbesondere in einem Umwälzbetrieb auf die Atemgeräte 112 aufgebracht werden, indem das Reinigungsfluid 132 aus dem Waschtank 134 über das Waschdüsensystem 126 auf die Atemgeräte 112 aufgesprüht und/oder aufgespritzt wird, um dann wieder in den Waschtank 134 abzulaufen oder abzutropfen, um von dort aus erneut verwendet zu werden. Optional können ein oder mehrere Filter, beispielsweise Grobfilter und/oder Feinfilter, vorgesehen sein, um das Reinigungsfluid 132 des Waschtanks 134 zumindest teilweise aufzureinigen.

Das Nachspüldüsensystem 128 kann beispielsweise über mindestens ein Nachspülleitungssystem 152 mit einem weiteren Reinigungsfluid 154, beispielsweise einer Nachspülflüssigkeit, beaufschlagt werden. Dabei ist in Figur 1 eine optionale Ausgestaltung gezeigt, bei welcher die Reinigungsvorrichtung 110 als Zweikreissystem ausgestaltet ist. Dementsprechend wird das zweite Reinigungsfluid 154 aus einem separaten Tank bereitgestellt, welcher in dem dargestellten Ausführungsbeispiel als Nachspültank 156 ausgestaltet ist, der getrennt von dem Waschtank 134 ausgebildet ist. Beispielsweise kann dieser Nachspültank 156 als Boiler ausgestaltet sein und kann beispielsweise eine Nachspültankheizung 158 umfassen. Alternativ oder zusätzlich zu einer Nachspültankheizung 158 können auch andere Arten von Heizelementen für das zweite Reinigungsfluid 154 vorgesehen sein, beispielsweise ein oder mehrere Durchlauferhitzer. Selbiges gilt auch für das erste Reinigungsfluid 132 in dem Waschtank 134. Wiederum können in dem Nachspültank 156 ein oder mehrere Temperatursensoren 160 und/oder ein oder mehrere Niveausensoren 162 vorgesehen sein, und der Nachspültank 156 kann über einen oder mehrere Zuläufe 164 mit Reinigungsfluid 154 beschickt werden, beispielsweise Frischwasser. Der mindestens eine Zulauf 164 kann ein oder mehrere Ventile 166 aufweisen. Beispielsweise kann der Zulauf 164 mit einem bauseitigen Frischwasseranschluss verbunden oder verbindbar sein. Alternativ oder zusätzlich kann, bauseitig oder als Bestandteil der Reinigungsvorrichtung 110, mindestens eine Umkehrosmosevorrichtung 170 vorgesehen sein, über welche der Nachspültank 156 und/oder ein oder mehrere andere Tanks der Reinigungsvorrichtung 110 mit einem Permeat, beispielsweise aufgereinigtem Wasser, beaufschlagt werden können. Weiterhin kann wiederum mindestens ein Dosiersystem 168 vorgesehen sein, über welches ein oder mehrere Zusätze zu dem Reinigungsfluid 154 in dem Nachspültank 156 beigemischt werden können, beispielsweise ein oder mehrere Klarspülerkonzentrate.

Die Beaufschlagung des Nachspüldüsensystems 128 kann vorzugsweise im einfachen Betrieb erfolgen, also nicht im Umwälzbetrieb, so dass das Nachspülfluid aus dem Nachspültank 154 das Atemgerät 112 lediglich einmal beaufschlagt. Zur Beaufschlagung kann die Fluideinrichtung 122 beispielsweise ein oder mehrere Drucksteigerungspumpen 172 umfassen.

Die Reinigung der Atemgeräte 112 in der Reinigungsvorrichtung 110 gemäß Figur 1 kann beispielsweise dadurch erfolgen, dass zunächst ein oder mehrere Atemgeräte 112 mittels einer geeigneten Halterung 174, welche unten noch näher erläutert wird, in die Reinigungskammer 116 eingebracht werden. Anschließend kann die Tür 120 verschlossen werden, und es kann vorzugsweise ein Reinigungsprogramm gestartet werden, welches beispielsweise über eine Steuerung 176, beispielsweise eine zentrale Maschinensteuerung oder eine dezentrale Steuerung, gesteuert werden kann. Dabei kann beispielsweise zunächst der Waschtank 136 mittels des Nachspüldüsensystems 128 mit Reinigungsfluid 132 und/oder einer Vorstufe dieses Reinigungsfluids 132 befüllt werden, beispielsweise Frischwasser, insbesondere demineralisiertem Frischwasser. Dieses kann dann innerhalb des Waschtanks 134 konditioniert werden, beispielsweise durch Beimengung von einem oder mehreren Zusätzen über das Dosiersystem 150 und/oder durch Erwärmung mittels des Heizelements 138. Alternativ oder zusätzlich kann das Reinigungsfluid 132 auch nach einem Klarspülprogramm eines vorangehenden Reinigungszyklus im Waschtank 134 verblieben sein, um in einem sich anschließenden Reinigungszyklus als Reinigungsfluid 132 und/oder als Bestandteil desselben genutzt werden, da Nachspülfluid in der Regel auch nach Beaufschlagung des Atemgeräts 112 einen vergleichsweise hohen Reinheitsgrad aufweist.

Anschließend kann das Atemgerät 112, vorzugsweise im Umwälzbetrieb, in einem oder mehreren Waschprogrammschritten gereinigt, insbesondere gewaschen, werden. Hierbei können anhaftende Verunreinigungen von den Atemgeräten 112 entfernt werden, und/oder es kann eine Hygienisierung der Atemgeräte 112 erfolgen.

Anschließend an den mindestens einen Waschprogrammschritt können vorzugsweise ein oder mehrere Nachspülschritte durchgeführt werden. Hierzu kann der Waschtank 134 über die Ablaufleitung 144 und die Ablaufpumpe 146 optional entleert werden. Bereits während des mindestens einen Waschprogrammschritts kann der Nachspültank 156 mit dem Nachspülfluid 154, beispielsweise Frischwasser mit oder ohne Zusätze, befüllt worden sein, beispielsweise demineralisiertem Frischwasser. Anschließend können eine oder mehrere Zusätze über das Dosiersystem 150 beigemischt werden und/oder es kann eine Erwärmung des Reinigungsfluids 154 als Nachspülfluid mittels der Nachspültankheizung 158 und/oder eines Durchlauferhitzers erfolgen. Das derart vorkonditionierte Nachspülfluid 154 kann dann in dem mindestens einen Nachspülschritt über das Nachspüldüsensystem 128 auf die Atemgeräte 112 aufgebracht werden, so dass diese nachgespült und/oder klargespült werden. Nach dem mindestens einen Nachspülschritt kann sich optional wiederum mindestens ein Trocknungsschritt anschließen, welcher passiv ausgestaltet werden kann, durch einfaches Abwarten, oder welcher auch aktiv unterstützt werden kann, beispielsweise über mindestens ein Trocknungsgebläse und/oder eine andere Art von Trocknungsvorrichtung der Reinigungsvorrichtung 110, beispielsweise ein Infrarotstrahlersystem und/oder ein Mikrowellenstrahlungssystem. Verschiedene Ausgestaltungen sind denkbar. Anschließend an den optionalen Trocknungsschritt kann die bis dahin optional vorzugsweise verriegelte Tür 120 automatisch freigegeben und/oder geöffnet werden. Der gesamte Programmablauf kann beispielsweise durch die Steuerung 176 gesteuert werden, wobei auch mehrere Programmabläufe wählbar sein können.

Es wird darauf hingewiesen, dass das in Figur 1 dargestellte Ausführungsbeispiel der Reinigungsvorrichtung 110 lediglich eines von mehreren verschiedenen Ausfiihrungsbeispielen darstellt. So können einzelne oder mehrere oder auch alle der oben beschriebenen Elemente auch in anderem Rahmen umgesetzt sein. Die Spülkammer 118 kann, alternativ oder zusätzlich zu der starren, ortsfesten Ausgestaltung gemäß Figur 1, auch schwenkbar und/oder drehbar ausgestaltet werden. Weiterhin kann auch der dargestellte Fluidkreislauf erheblich modifiziert werden.

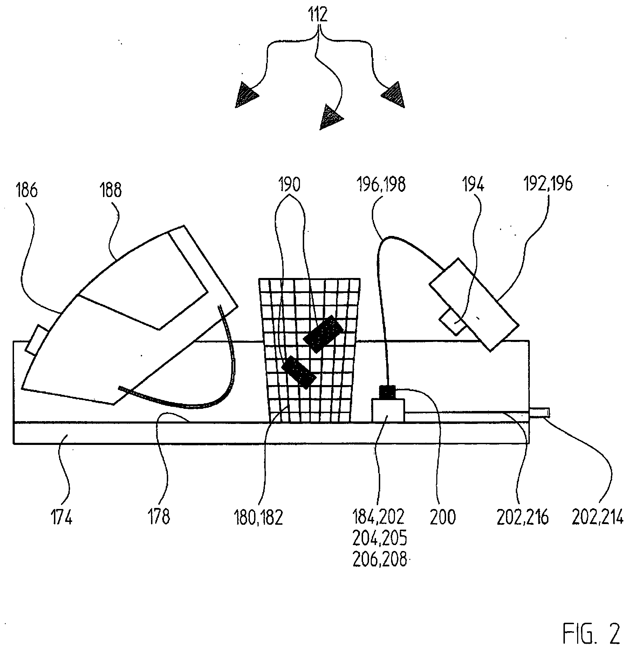

Wie oben dargestellt, wird das mindestens eine Atemgerät 112 innerhalb der Reinigungsvorrichtung 110 mittels mindestens einer Halterung 174 gehaltert. Eine derartige Halterung 174 ist in vergrößerter Darstellung in Figur 2 in einer Schnittdarstellung exemplarisch gezeigt. Die Halterung 174 kann beispielsweise aus Kunststoff und/oder Metall hergestellt sein und kann beispielsweise, wie in Figur 2 gezeigt, als Korb ausgestaltet werden. Die Halterung 174 kann eine oder mehrere Auflageflächen 178 und/oder ein oder mehrere Halteelemente 180 umfassen, beispielsweise einen oder mehrere Körbe 182 und/oder ein oder mehrere Verbindungselemente 184 für eine mechanische Verbindung mit dem Atemgerät 112 und/oder dessen Fixierung. Dementsprechend kann die Halterung 174 beispielsweise ausgestaltet sein, um eine oder mehrere Komponenten des Atemgeräts 112 relativ zu einer oder mehreren der Düsen 124 auszurichten und/oder zu fixieren. So kann das Atemgerät 112 beispielsweise mindestens eine Atemmaske 186 umfassen, wobei die Ausrichtung durch die Auflagefläche 178 beispielsweise derart erfolgen kann, dass stets eine Sichtscheibe 188 dem oberen Düsensystem zuweist, wohingegen die Innenseite der Atemmaske 186 nach unten weist, so dass über die unteren Düsen eintretendes Reinigungsfluid 132, 154 nach der Beaufschlagung wieder ablaufen kann, ohne dass Reste innerhalb der Atemmaske 186 verbleiben. Der Korb 182 kann beispielsweise eingerichtet sein, um Zubehör 190, welches ebenfalls Bestandteil des Atemgeräts 112 sein kann, aufzunehmen, beispielsweise um eine Zuordnung zu anderen Komponenten des Atemgeräts 112 während des Reinigungsvorgangs aufrechtzuerhalten. Auf diese Weise kann beispielsweise gewährleistet werden, dass unterschiedliche Arten von Atemgeräten 112 gereinigt werden können, ohne dass inkompatible Zubehörteile vermischt werden. Weiterhin kann das Atemgerät 112 beispielsweise einen oder mehrere Lungenautomaten 192 und/oder eine oder mehrere Stufen derartiger Lungenautomaten 192 umfassen. Derartige Lungenautomaten 192 umfassen in der Regel in ihrem Inneren ein oder mehrere Ventile 194, beispielsweise Membran- und/oder Kipphebelventile, über welche ein oder mehrere gasführende Elemente 196 von einem Außenraum abgetrennt sind. Beispielsweise können gasführende Elemente 196 im Inneren einer oder mehrerer Gasleitungen 198 des Lungenautomaten 192, vorgesehen sein, wobei die Innenwände der Gasleitung 198 in der Regel nicht mit Reinigungsfluid 132, 154 in Kontakt kommen dürfen. Die Gasleitung 198 kann beispielsweise in einem oder mehreren Anschlüssen 200 enden, beispielsweise in einer oder mehreren Schnellkupplungen.

Um erfindungsgemäß zu verhindern, dass das mindestens eine gasführende Element 196 der Atemgeräte 112, beispielsweise die Gasleitung 198 des Lungenautomaten 192, mit Reinigungsfluid 132, 154 in Kontakt kommt, wird erfindungsgemäß vorgesehen, dieses gasführende Element 196 mit Druckgas zu beaufschlagen, beispielsweise während eines oder mehrere Programmschritte, in denen das Atemgerät 112 mit dem Reinigungsfluid 132, 154 beaufschlagt wird. Zu diesem Zweck weist die Reinigungsvorrichtung 110 in diesem oder auch in anderen Ausführungsbeispielen der Erfindung mindestens eine Druckbeaufschlagungsvorrichtung 202 auf. Diese Druckbeaufschlagungsvorrichtung 202 ist eingerichtet, um das mindestens eine gasführende Element 196 mit Druckgas zu beaufschlagen. Zu diesem Zweck weist die Druckbeaufschlagungsvorrichtung 202 mindestens einen Druckanschluss 204 auf, welcher mit dem gasführenden Element 196 derart verbindbar ist, dass eine derartige Gasbeaufschlagung erfolgen kann. Dieser Druckanschluss 204 ist in dem dargestellten Ausführungsbeispiel exemplarisch als Bestandteil der Halterung 174 ausgebildet. Alternativ oder zusätzlich kann der Druckanschluss 204 jedoch auch an anderen Stellen der Reinigungsvorrichtung 110, im Inneren der Reinigungskammer 116vorgesehen sein. Der Druckanschluss 204 kann beispielsweise in dem dargestellten Ausführungsbeispiel als Anschluss 206 für den Lungenautomaten 192 ausgestaltet sein. Der Druckanschluss 204 kann dabei allgemein in diesem oder auch in anderen Ausführungsbeispielen derart ausgestaltet sein, dass dieser an mehrere Arten gasführender Elemente 196, beispielsweise an unterschiedliche Typen und/oder unterschiedliche Arten gasführender Elemente 196, anschließbar ist. Beispielsweise kann der Druckanschluss 204 eine Mehrzahl von Adaptern 205 umfassen und/oder die Reinigungsvorrichtung 110 kann mit einem Adaptersatz mit mehreren Adaptern 205 verschiedener Druckanschlüsse 204 geliefert werden, so dass eine hohe Flexibilität hinsichtlich der Art der gasführenden Elemente 196 besteht. Beispielsweise kann der Anschluss 206 für den Lungenautomaten 192 eine Schnellkupplung umfassen, welche zu dem Anschluss 200 der Gasleitung 198 korrespondiert und mit dieser vorzugsweise druckdicht verbindbar ist. In diesem oder auch in anderen Fällen kann der Druckanschluss 204 somit auch mindestens ein Verbindungselement 208 umfassen und/oder als Verbindungselement 208 zur Herstellung einer mechanischen Verbindung mit dem Atemgerät 112 und insbesondere dem gasführenden Element 196 ausgestaltet sein.

Zur Beaufschlagung des Druckanschlusses 204 und des gasführenden Elements 196 mit Druckgas, beispielsweise Druckluft, kann die Reinigungsvorrichtung 110 in dem dargestellten Ausführungsbeispiel oder auch in anderen Ausgestaltungen mindestens eine interne und/oder mindestens eine externe Druckgasquelle umfassen. Eine optionale integrierte Druckgasquelle 209, welche in Figur 1 angedeutet ist, kann beispielsweise mindestens einen Kompressor und/oder mindestens eine Druckgasflasche umfassen. Alternativ oder zusätzlich kann die Reinigungsvorrichtung 110, wie in Figur 1 dargestellt, auch über mindestens einen externen Druckanschluss 210 ausgestattet sein, um über diesen externen Druckanschluss 210 mit mindestens einer externen Druckgasquelle verbunden zu sein, beispielsweise einer externen Druckgasflasche und/oder einer externen Druckgasleitung, welche beispielsweise gebäudeseitig vorgesehen sein kann. Der externe Druckanschluss 210 und/oder die interne Druckgasquelle können beispielsweise über mindestens eine Druckgasleitung 212 mit dem Druckanschluss 204 verbunden sein. Ist der Druckanschluss 204 mit einer Halterung 174 verbunden, wie in den Figuren 1 und 2 dargestellt, so kann die Druckgasleitung 212 beispielsweise lösbar mit der Halterung 174 und/oder dem Druckanschluss 204 verbunden sein, um optional ein reversibles Herausnehmen der Halterung 174 aus der Reinigungsvorrichtung 110 zu ermöglichen. Beispielsweise können zu diesem Zweck eine oder mehrere Kupplungen 214 vorgesehen sein, welche beispielsweise wiederum über eine oder mehrere Druckgasleitungen 216 mit dem Druckanschluss 204 verbunden sein können. Auch ein Verteilersystem kann innerhalb oder außerhalb der Halterung 174 vorgesehen sein, beispielsweise um mittels einer Druckgasleitung 212 eine Mehrzahl von Druckanschlüssen 204 mit Druckgas beaufschlagen zu können. Wiederum können auch eine oder mehrere Adapter 205 vorgesehen sein, beispielsweise um eine Anpassung auf eine Mehrzahl unterschiedlicher Arten von gasführenden Elementen 196 und/oder Atemgeräten 112 zu ermöglichen. Die Druckbeaufschlagungsvorrichtung 202 kann an einer oder mehreren Stellen trennbar ausgestaltet sein, beispielsweise um die Halterung 174 aus der Reinigungskammer 116 entfernen und beispielsweise bestücken zu können. Zu diesem Zweck kann beispielsweise die Kupplung 214 lösbar ausgestaltet sein. Weiterhin kann die Druckbeaufschlagungsvorrichtung 202 in diesem oder auch in anderen Ausführungsbeispielen der erfindungsgemäßen Reinigungsvorrichtung 110 optional ein oder mehrere Ventile 218 umfassen, beispielsweise steuerbare Ventile, beispielsweise (wie in Figur 1 dargestellt) in der Druckgasleitung 212 und/oder in oder vorgeschaltet zu dem externen Druckanschluss 210. Diese Ventile 218 können beispielsweise durch die Steuerung 176 gesteuert werden. So kann beispielsweise auf diese oder auch auf andere Weise die Druckbeaufschlagung des mindestens einen gasführenden Elements 196 mittels der Druckbeaufschlagungsvorrichtung 202 in diesem oder auch in anderen Ausführungsbeispielen der Erfindung gesteuert erfolgen, beispielsweise indem die Druckbeaufschlagung gezielt vor Beginn einer Beaufschlagung der Atemgeräte 112 mit Reinigungsfluid 132, 154 erfolgt. Alternativ oder zusätzlich kann die Druckbeaufschlagung auch lediglich in einem oder mehreren Programmschritten erfolgen, beispielsweise lediglich in einem oder mehreren Programmschritten während einer Beaufschlagung der Atemgeräte 112 mit Reinigungsfluid 132, 154. Optional kann die Beaufschlagung mit Druckgas, beispielsweise Druckluft, während anderer Programmschritte hingegen abgeschaltet werden und/oder in anderer Form erfolgen, beispielsweise mit verändertem Druck. Auf diese Weise kann die Druckbeaufschlagung beispielsweise den verschiedenen Programmschritten angepasst werden.

**Bezugszeichenliste**

| | | | |
|---|---|---|---|
| 110 | Reinigungsvorrichtung | 178 | Auflagefläche |
| 112 | Atemgeräte | 180 | Halteelement |
| 114 | Spülmaschine | 182 | Korb |
| 116 | Reinigungskammer | 184 | Verbindungselement |
| 118 | Spülkammer | 186 | Atemmaske |
| 120 | Tür | 188 | Sichtscheibe |
| 122 | Fluideinrichtung | 190 | Zubehör |
| 124 | Düsen | 192 | Lungenautomat |
| 126 | Waschdüsensystem | 194 | Ventil |
| 128 | Nachspüldüsensystem | 196 | gasführendes Element |
| 130 | Waschleitungssystem | 198 | Gasleitung |
| 132 | Reinigungsfluid, Waschfluid | 200 | Anschluss |
| 134 | Waschtank | 202 | Druckbeaufschlagungsvorrichtung |
| 136 | Umwälzpumpe | 204 | Druckanschluss |
| 138 | Heizelement | 205 | Adapter |
| 140 | Temperatursensor | 206 | Anschluss für Lungenautomat |
| 142 | Niveausensor | 208 | Verbindungselement |
| 144 | Ablaufleitung | 209 | integrierte Druckgasquelle |
| 146 | Ablaufpumpe | 210 | externer Druckanschluss |
| 148 | Ablauf | 212 | Druckgasleitung |
| 150 | Dosiersystem | 214 | Kupplung |
| 152 | Nachspülleitungssystem | 216 | Druckgasleitung |
| 154 | Reinigungsfluid, Nachspülfluid | 218 | Ventile |
| 156 | Nachspültank | | |
| 158 | Nachspültankheizung | | |
| 160 | Temperatursensor | | |
| 162 | Niveausensor | | |
| 164 | Zulauf | | |
| 166 | Ventil | | |
| 168 | Dosiersystem | | |
| 170 | Umkehrosmosevorrichtung | | |
| 172 | Drucksteigerungspumpe | | |
| 174 | Halterung | | |
| 176 | Steuerung | | |

## Patentansprüche

1. Reinigungsvorrichtung (110) zur Reinigung von Atemgeräten (112), umfassend mindestens eine Reinigungskammer (116) zur Aufnahme mindestens eines Atemgeräts (112), wobei die Reinigungsvorrichtung (110) weiterhin mindestens eine Fluideinrichtung (122) zur Beaufschlagung des Atemgeräts (112) mit mindestens einem Reinigungsfluid (132, 154) aufweist, wobei das Reinigungsfluid eine Flüssigkeit ist, wobei die Fluideinrichtung (122) mindestens eine Düse (124) aufweist, wobei die Beaufschlagung in Form eines direkten Beaufschlagens erfolgt, wobei aus der Fluideinrichtung (122) austretendes Reinigungsfluid unmittelbar auf das Atemgerät (112) auftrifft, wobei die Beaufschlagung ausgewählt ist aus einem Besprühen, Betropfen, Bestrahlen oder eine Kombination der genannten Beaufschlagungsarten, wobei das Atemgerät (112) während der Beaufschlagung nicht in das Reinigungsfluid eintaucht, so dass das Reinigungsfluid unmittelbar bei oder unmittelbar nach dem Auftreffen auf das Atemgerät (112) wieder von diesem abfließen und/oder abtropfen kann, wobei die Reinigungsvorrichtung (110) mindestens eine in die Reinigungskammer (116) einbringbare Halterung (174) zur Aufnahme des Atemgeräts (112) aufweist, wobei die Reinigungsvorrichtung (110) als Spülmaschine (114) ausgestaltet ist, bei welcher das Atemgerät (112) in der Halterung (174) aufgenommen ist und wobei die Fluideinrichtung (122) jeweils mindestens eine Düse (124) oberhalb und mindestens eine Düse (124) unterhalb der Halterung (174) aufweist, wobei nach der Beaufschlagung das Reinigungsfluid von dem Atemgerät (112) in einen Waschtank (134) abfließen und/oder abtropfen kann, wobei die Halterung (174) eingerichtet ist, um das Atemgerät (112) relativ zu der Fluideinrichtung (122) auszurichten, wobei die Reinigungsvorrichtung (110) weiterhin mindestens eine Druckbeaufschlagungsvorrichtung (202) mit mindestens einem Druckanschluss (204) aufweist, wobei der Druckanschluss (204) mit mindestens einem gasführenden Element (196) des Atemgeräts (112) verbindbar ist,
**dadurch gekennzeichnet, dass**
die Druckbeaufschlagungsvorrichtung (202) eingerichtet ist, um das gasführende Element (196) während der Beaufschlagung mit dem Reinigungsfluid (132, 154) mit Druckgas zu beaufschlagen.

2. Reinigungsvorrichtung (110) nach dem vorhergehenden Anspruch, wobei der Druckanschluss (204) mindestens ein Verbindungselement (208) umfasst, um zusätzlich eine mechanische Verbindung mit dem Atemgerät (112) herzustellen, wobei das Verbindungselement (208) ausgewählt ist aus der Gruppe bestehend aus einem formschlüssigen Verbindungselement und einem kraftschlüssigen Verbindungselement (208).

3. Reinigungsvorrichtung (110) nach einem der vorhergehenden Ansprüche, wobei der Druckanschluss (204) eine Mehrzahl unterschiedlicher Adapter (205) zur Verbindung mit unterschiedlichen Arten gasführender Elemente (196) aufweist.

4. Reinigungsvorrichtung (110) nach einem der vorhergehenden Ansprüche, wobei die Druckbeaufschlagungsvorrichtung (202) mindestens eines der folgenden Elemente aufweist: einen externen Druckanschluss (210) zur Verbindung mit einer externen Druckgasquelle; mindestens eine in die Reinigungsvorrichtung (110) integrierte Druckgasquelle.

5. Reinigungsvorrichtung (110) nach einem der vorhergehenden Ansprüche, wobei die Halterung (174) eine herausnehmbare Halterung (174) ist.

6. Reinigungsvorrichtung (110) nach dem vorhergehenden Anspruch, wobei der Druckanschluss (204) zumindest teilweise in die Halterung (174) integriert ist.

7. Reinigungsvorrichtung (110) nach einem der beiden vorhergehenden Ansprüche, wobei die Halterung (174) eine lösbare Druckgasverbindung (214) zur übrigen Reinigungsvorrichtung (110) aufweist.

8. Reinigungsvorrichtung (110) nach einem der vorhergehenden Ansprüche, wobei die Reinigungsvorrichtung (110) eine Einkammerspülmaschine umfasst, wobei die Einkammerspülmaschine mindestens einen separat von der Reinigungskammer (116) ausgebildeten Fluidtank (156) aufweist, wobei in dem Fluidtank (156) mindestens ein Reinigungsfluid (132, 154) unabhängig von einem in der Reinigungsvorrichtung (110) ablaufenden Reinigungsprozess konditionierbar, insbesondere erwärmbar, ist.

9. Reinigungsvorrichtung (110) nach einem der vorhergehenden Ansprüche, wobei die Reinigungsvorrichtung (110) eingerichtet ist, um ein Reinigungsprogramm mit mindestens zwei unterschiedlichen Programmschritten durchzuführen.

10. Reinigungsvorrichtung (110) nach einem der vorhergehenden Ansprüche, wobei die Düse (124) ausgewählt ist aus der Gruppe bestehend aus: einer Sprühdüse; einer Spüldüse; einem Sprüharm, insbesondere einem drehbaren Sprüharm; einer im Umwälzbetrieb betreibbaren Düse.

11. Reinigungsvorrichtung (110) nach einem der vorhergehenden Ansprüche, wobei das Reinigungsfluid (132, 154) ausgewählt ist aus der Gruppe bestehend aus: einem wässrigen Reinigungsfluid (132, 154); einem Reinigungsfluid (132, 154) mit mindestens einer Reinigerlösung; einem Reinigungsfluid (132, 154) mit mindestens einem Klarspüler; einem Reinigungsfluid (132, 154) mit mindestens einem Desinfektionsmittel; einem Nachspülfluid; demineralisiertem Wasser; einem erwärmten Reinigungsfluid (132, 154), insbesondere einem auf eine Temperatur von 30 °C bis 70 °C und besonders bevorzugt von 60 °C erwärmtem Reinigungsfluid (132, 154).

12. Reinigungsvorrichtung (110) nach einem der vorhergehenden Ansprüche, wobei die Reinigungsvorrichtung (110) mindestens eine Umkehrosmosevorrichtung (170) zur Bereitstellung von Wasser, insbesondere demineralisiertem Wasser, aufweist.

13. Verwendung der Reinigungsvorrichtung (110) nach Anspruch 1, für eine Reinigung mindestens eines Atemgeräts (112).

14. Verwendung nach dem vorhergehenden Anspruch, wobei das Atemgerät (112) ausgewählt ist aus der Gruppe bestehend aus: einem Lungenautomaten (192) für Taucher; einer Atemmaske (186) für Taucher; einem Lungenautomaten (192) für Rettungskräfte; einer Atemschutzmaske (186) für Rettungskräfte; einem Pressluftatmer; einem Lungenautomaten (192) für Polizei; einem Lungenautomaten (192) für Streitkräfte; einer Atemmaske (186) für Polizei; einer Atemmaske (186) für Streitkräfte; einem Lungenautomaten (192) für medizinische Zwecke; einer Atemmaske (186) für medizinische Zwecke.

15. Verfahren zur Reinigung eines Atemgeräts (112), umfassend eine Verwendung der Reinigungsvorrichtung (110) nach Anspruch 1.

## Claims

1. Cleaning device (110) for cleaning respirators (112), comprising at least one cleaning chamber (116) for receiving at least one respirator (112), wherein the cleaning device (110) moreover has at least one fluid device (122) for applying at least one cleaning fluid (132, 154) to the respirator (112), wherein the cleaning fluid is a liquid, wherein the fluid device (122) has at least one nozzle (124), wherein the application takes place in the form of a direct application, wherein cleaning fluid emerging from the fluid device (122) impinges directly on the respirator (112), wherein the application is chosen from spraying, sprinkling, jetting or a combination of said types of application, wherein the respirator (112) is not immersed in the cleaning fluid during the application, so that, immediately when or immediately after it impinges on the respirator (112), the cleaning fluid can flow and/or drip off from it again, wherein the cleaning device (110) has at least one holder (174), which can be inserted into the cleaning chamber (116) and receives the respirator (112), wherein the cleaning device (110) is designed as a washer (114) in which the respirator (112) is received in the holder (174) and wherein the fluid device (122) has in each case at least one nozzle (124) above and at least one nozzle (124) below the holder (174), wherein the cleaning fluid is able to flow and/or drip off from the respirator (112) into a washing tank (134) after being applied, wherein the holder (174) is designed to align the respirator (112) relative to the fluid device (122), wherein the cleaning device (110) moreover has at least one pressure application device (202) with at least one pressure attachment (204), wherein the pressure attachment (204) is connectable to at least one gas-carrying element (196) of the respirator (112),
**characterized in that**
the pressure application device (202) is designed to apply pressurized gas to the gas-carrying element (196) during the application of the cleaning fluid (132, 154).

2. Cleaning device (110) according to the preceding claim, wherein the pressure attachment (204) comprises at least one connection element (208), in order additionally to produce a mechanical connection to the respirator (112), wherein the connection element (208) is chosen from the group consisting of a form-fit connection element and a force-fit connection element (208).

3. Cleaning device (110) according to one of the preceding claims, wherein the pressure attachment (204) has a plurality of different adapters (205) for connecting to different types of gas-carrying elements (196).

4. Cleaning device (110) according to one of the preceding claims, wherein the pressure application device (202) has at least one of the following elements: an external pressure attachment (210) for connecting to an external source of pressurized gas; at least one source of pressurized gas integrated in the cleaning device (110).

5. Cleaning device (110) according to one of the preceding claims, wherein the holder (174) is a removable holder (174).

6. Cleaning device (110) according to the preceding claim, wherein the pressure attachment (204) is integrated at least partially in the holder (174).

7. Cleaning device (110) according to one of the preceding two claims, wherein the holder (174) has a releasable pressurized gas connection (214) to the rest of the cleaning device (110).

8. Cleaning device (110) according to one of the preceding claims, wherein the cleaning device (110) comprises a single-chamber washer, wherein the single-chamber washer has at least one fluid tank (156) formed separately from the cleaning chamber (116), wherein at least one cleaning fluid (132, 154) can be conditioned, in particular heated, in the fluid tank (156) independently of a cleaning process proceeding in the cleaning device (110).

9. Cleaning device (110) according to one of the preceding claims, wherein the cleaning device (110) is designed to perform a cleaning program with at least two different program steps.

10. Cleaning device (110) according to one of the preceding claims, wherein the nozzle (124) is chosen from the group consisting of: a spraying nozzle; a rinsing nozzle; a spraying arm, in particular a rotatable spraying arm; a nozzle that can be operated in a circulating mode.

11. Cleaning device (110) according to one of the preceding claims, wherein the cleaning fluid (132, 154) is chosen from the group consisting of: an aqueous cleaning fluid (132, 154); a cleaning fluid (132, 154) with at least one cleaning agent solution; a cleaning fluid (132, 154) with at least one rinse aid; a cleaning fluid (132, 154) with at least one disinfectant; a post-rinsing fluid; demineralized water; a heated cleaning fluid (132, 154), in particular a cleaning fluid (132, 154) heated to a temperature of 30°C to 70°C and particularly preferably of 60°C.

12. Cleaning device (110) according to one of the preceding claims, wherein the cleaning device (110) has at least one reverse osmosis device (170) for supplying water, in particular demineralized water.

13. Use of the cleaning device (110) according to Claim 1, for cleaning at least one respirator (112).

14. Use according to the preceding claim, wherein the respirator (112) is chosen from the group consisting of: a breathing regulator (192) for divers; a breathing mask (186) for divers; a breathing regulator (192) for members of rescue teams; a breathing mask (186) for members of rescue teams; a compressed air breathing apparatus; a breathing regulator (192) for the police; a breathing regulator (192) for military forces; a breathing mask (186) for the police; a breathing mask (186) for military forces; a breathing regulator (192) for medical purposes; a breathing mask (186) for medical purposes.

15. Method for cleaning a respirator (112), comprising use of the cleaning device (110) according to Claim 1.

## Revendications

1. Dispositif de nettoyage (110) pour le nettoyage d'appareils de respiration (112), comprenant au moins une chambre de nettoyage (116) destinée à contenir au moins un appareil de respiration (112), dans lequel le dispositif de nettoyage (110) présente en outre au moins un système de fluide (122) pour exposer l'appareil de respiration (112) à au moins un fluide de nettoyage (132, 154), dans lequel le fluide de nettoyage est un liquide, dans lequel le système de fluide (122) présente au moins une buse (124), dans lequel l'exposition est effectuée sous la forme d'une exposition directe, dans lequel un fluide de nettoyage sortant du système de fluide (122) frappe directement l'appareil de respiration (112), dans lequel l'exposition est choisie parmi une pulvérisation, une aspersion avec des gouttes, une projection ou une combinaison des modes d'exposition précités, dans lequel l'appareil de respiration (112) ne pénètre pas dans le fluide de nettoyage pendant l'exposition de sorte que le fluide de nettoyage peut à nouveau s'écouler et/ou s'égoutter de l'appareil de respiration (112) après avoir heurté celui-ci, dans lequel le dispositif de nettoyage (110) présente au moins un support (174) apte à être introduit dans la chambre de nettoyage (116) afin de recevoir l'appareil de respiration (112), dans lequel le dispositif de nettoyage (110) est conçu comme une machine de pulvérisation (114) dans laquelle l'appareil de respiration (112) est reçu dans le support (174) et dans lequel le dispositif de respiration (122) comporte au moins une buse (124) au-dessus du support (174) et au moins une buse (124) au-dessous du support (174), dans lequel le fluide de nettoyage peut s'écouler et/ou s'égoutter, après l'exposition, de l'appareil de respiration (112) jusque dans un réservoir de lavage (134), dans lequel le support (174) est conçu pour aligner le dispositif de respiration (112) par rapport au système de fluide (122), dans lequel le dispositif de nettoyage (110) présente en outre au moins un dispositif d'exposition sous pression (202) avec au moins un raccord de pression (204), dans lequel le raccord de pression (204) peut être raccordé à au moins un élément de conduite de gaz (196) de l'appareil de respiration (112),
**caractérisé en ce que** le dispositif d'exposition sous pression (202) est conçu pour alimenter l'élément de conduite de gaz (196) avec un gaz sous pression pendant l'exposition avec le fluide de nettoyage (132, 154).

2. Dispositif de nettoyage (110) selon la revendication précédente, dans lequel le raccord de pression (204) comprend au moins un élément de liaison (208), afin de réaliser en plus une liaison mécanique avec l'appareil de respiration (112), dans lequel l'élément de liaison (208) est choisi dans le groupe composé de : un élément de liaison par emboîtement et un élément de liaison par adhérence (208).

3. Dispositif de nettoyage (110) selon l'une quelconque des revendications précédentes, dans lequel le raccord de pression (204) présente une multiplicité d'adaptateurs différents (205) en vue de la liaison à différents types d'éléments de conduite de gaz (196).

4. Dispositif de nettoyage (110) selon l'une quelconque des revendications précédentes, dans lequel le dispositif d'exposition sous pression (202) présente au moins un des éléments suivants : un raccord de pression extérieur (210) pour la liaison à une source de gaz sous pression externe ; au moins une source de gaz sous pression intégrée dans le dispositif de nettoyage (110).

5. Dispositif de nettoyage (110) selon l'une quelconque des revendications précédentes, dans lequel le support (174) est un support (174) pouvant être extrait.

6. Dispositif de nettoyage (110) selon la revendication précédente, dans lequel le raccord de pression (204) est au moins partiellement intégré dans le support (174).

7. Dispositif de nettoyage (110) selon l'une quelconque des deux revendications précédentes, dans lequel le support (174) présente une liaison de gaz sous pression séparable (214) avec le reste du dispositif de nettoyage (110).

8. Dispositif de nettoyage (110) selon l'une quelconque des revendications précédentes, dans lequel le dispositif de nettoyage (110) présente une machine de rinçage à une chambre, dans lequel la machine de rinçage à une chambre présente au moins un réservoir de fluide (156) formé séparément de la chambre de nettoyage (116), dans lequel au moins un fluide de nettoyage (132, 154) peut être conditionné, en particulier peut être chauffé dans le réservoir de fluide (156), indépendamment d'un processus de nettoyage qui se déroule dans le dispositif de nettoyage (110).

9. Dispositif de nettoyage (110) selon l'une quelconque des revendications précédentes, dans lequel le dispositif de nettoyage (110) est conçu pour exécuter un programme de nettoyage avec au moins deux étapes de programme différentes.

10. Dispositif de nettoyage (110) selon l'une quelconque des revendications précédentes, dans lequel la buse (124) est choisie dans le groupe composé de : une buse de pulvérisation ; une buse de rinçage ; un bras de pulvérisation, en particulier un bras de pulvérisation rotatif ; une buse utilisable en mode de recirculation.

11. Dispositif de nettoyage (110) selon l'une quelconque des revendications précédentes, dans lequel le fluide de nettoyage (132, 154) est choisi dans le groupe composé de : un fluide de nettoyage aqueux (132, 154) ; un fluide de nettoyage (132, 154) avec au moins une solution d'un agent nettoyant ; un fluide de nettoyage (132, 154) avec au moins un agent mouillant ; un fluide de nettoyage (132, 154) avec au moins un agent de désinfection ; un fluide de rinçage ; l'eau déminéralisée ; un fluide de nettoyage (132, 154) chauffé, en particulier un fluide de nettoyage (132, 154) chauffé à une température de 30°C à 70°C et en particulier de préférence de 60°C.

12. Dispositif de nettoyage (110) selon l'une quelconque des revendications précédentes, dans lequel le dispositif de nettoyage (110) présente au moins un dispositif d'osmose inverse (170) pour la préparation d'eau, en particulier d'eau déminéralisée.

13. Utilisation du dispositif de nettoyage (110) selon la revendication 1, pour un nettoyage d'au moins un appareil de respiration (112).

14. Utilisation selon la revendication précédente, dans laquelle l'appareil de respiration (112) est choisi dans le groupe composé de : un régulateur respiratoire (192) pour plongeurs ; un masque respiratoire (186) pour plongeurs ; un régulateur respiratoire (192) pour services de sauvetage ; un masque de protection respiratoire (186) pour services de sauvetage ; un respirateur à air comprimé ; un régulateur respiratoire (192) pour la police ; un régulateur respiratoire (192) pour les forces armées ; un masque respiratoire (186) pour la police ; un masque respiratoire (186) pour les forces armées ; un régulateur respiratoire (192) pour des applications médicales ; un masque respiratoire (186) pour des applications médicales.

15. Procédé de nettoyage d'un appareil de respiration (112), comprenant une utilisation du dispositif de nettoyage (110) selon la revendication 1.
